# EUROPEAN PATENT SPECIFICATION

(11) **EP 1 062 341 B1**
(45) Date of publication and mention of the grant of the patent: **17.09.2008**
(21) Application number: 99907345.5
(22) Date of filing: 16.03.1999
(51) Int. Cl.: C12N 15/29, C12N 15/12, C07K 14/415, C07K 14/435, A61K 39/35, A61K 39/36

(54) **MUTANT RECOMBINANT ALLERGENS**
MUTIERTES REKOMBINANTES ALLERGEN
ALLERGENES RECOMBINES MUTANTS

(30) Priority: 16.03.1998 DK 36498
(43) Date of publication of application: 27.12.2000
(73) Proprietor: ALK-Abelló A/S, 2970 Hørsholm (DK)
(72) Inventor: IPSEN, Hans, Henrik, DK-3400 Hillerød (DK); SPANGFORT, Michael, Dho, S-25591 Helsingborg (SE); LARSEN, Jørgen Nedergaard, DK-3230 Graested (DK)
(74) Representative: Christiansen, Ejvind
(86) International application number: PCT/DK1999/000136
(87) International publication number: WO 1999/047680

(56) References cited:
- WO-A-97/33910
- FERREIRA F ET AL.: "Modulation of IgE reactivity of allergens by site-directed mutagenesis: potential use of hypoallergenic variants for immunotherapy" FASEB JOURNAL FOR EXPERIMENTAL BIOLOGY, vol. 12, no. 2, February 1998 (1998-02), pages 231-242, XP002085249 BETHESDA, MD US cited in the application
- WIEDEMANN P ET AL.: "Molecular and structural analysis of a continuous birch profilin epitope defined by a monoclonal antibody" JOURNAL OF BIOLOGICAL CHEMISTRY, vol. 271, no. 47, 22 November 1996 (1996-11-22), pages 29915-29921, XP002085250 MD US cited in the application
- SMITH A M AND CHAPMAN M D: "Localization of antigenic sites on Der p 2 using oligonucleotide-directed mutagenesis targeted to predicted surface residues" CLINICAL AND EXPERIMENTAL ALLERGY, vol. 27, no. 5, May 1997 (1997-05), pages 593-599, XP002085251 cited in the application
- SPANGFORT M D ET AL.: "Three-dimensional structure and epitopes of Bet v 1" INTERNATIONAL ARCHIVES OF ALLERGY AND IMMUNOLOGY, vol. 113, no. 1-3, 1997, pages 243-245, XP002085253
- HOFFMAN D R: "ALLERGENS IN HYMENOPTERA VENOM XXV: THE AMINO ACID SEQUENCES OF ANTIGEN 5 MOLECULES AND THE STRUCTURAL BASIS OF ANTIGENIC CROSS -REACTIVITY" JOURNAL OF ALLERGY AND CLINICAL IMMUNOLOGY, vol. 92, no. 5, 1 November 1993 (1993-11-01), pages 707-716, XP002035181 ISSN: 0091-6749

### SEQUENCE LISTING

<110> ALK Abello A/S
<120> Novel recombinant allergens
<130> P199800379 WO PCTDK9900136
<140> PCTDK9900136
   <141> 1999-03-16
<150> DK 0364/98
   <151> 1998-03-16
<160> 12
<170> PatentIn Ver. 2.1
<210> 1
   <211> 159
   <212> PRT
   <213> Artificial Sequence
<220>
   <223> Description of Artificial Sequence: Modified Bet v 1 sequence, Thr10Pro modification
<400> 1
   Gly Val Phe Asn Tyr Glu Thr Glu Thr Pro Ser Val Ile Pro Ala Ala
    1               5                  10                  15
   Arg Leu Phe Lys Ala Phe Ile Leu Asp Gly Asp Asn Leu Phe Pro Lys
               20                  25                  30
   Val Ala Pro Gln Ala Ile Ser Ser Val Glu Asn Ile Glu Gly Asn Gly
           35                  40                  45
   Gly Pro Gly Thr Ile Lys Lys Ile Ser Phe Pro Glu Gly Leu Pro Phe
       50                  55                  60
   Lys Tyr Val Lys Asp Arg Val Asp Glu Val Asp His Thr Asn Phe Lys
   65                  70                  75                  80
   Tyr Asn Tyr Ser Val Ile Glu Gly Gly Pro Ile Gly Asp Thr Leu Glu
                   85                  90                  95
   Lys Ile Ser Asn Glu Ile Lys Ile Val Ala Thr Pro Asp Gly Gly Ser
               100                 105                 110
   Ile Leu Lys Ile Ser Asn Lys Tyr His Thr Lys Gly Asp His Glu Val
           115                 120                 125
   Lys Ala Glu Gln Val Lys Ala Ser Lys Glu Met Gly Glu Thr Leu Leu
       130                 135                 140
   Arg Ala Val Glu Ser Tyr Leu Leu Ala His Ser Asp Ala Tyr Asn
   145                 150                 155
<210> 2
   <211> 159
   <212> PRT
   <213> Artificial Sequence
<220>
   <223> Description of Artificial Sequence: Modified Bet v 1 sequence, Asn28Thr + Lys32Gln modification
<400> 2
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Thr Leu Phe Pro Gln
   20 25 30
   Val Ala Pro Gln Ala Ile Ser Ser Val Glu Asn Ile Glu Gly Asn Gly
   35 40 45
   Gly Pro Gly Thr Ile Lys Lys Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 3
   <211> 159
   <212> PRT
   <213> Artificial Sequence
<220>
   <223> Description of Artificial Sequence: Modified Bet v 1 sequence, Glu45Ser modification
<400> 3
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Asn Leu Phe Pro Lys
   20 25 30
   Val Ala Pro Gln Ala Ile Ser Ser Val Glu Asn Ile Ser Gly Asn Gly
   35 40 45
   Gly Pro Gly Thr Ile Lys Lys Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 4
   <211> 159
   <212> PRT
   <213> Artificial Sequence
<220>
   <223> Description of Artificial Sequence: Modified Bet v 1 sequence, Lys55Asn modification
<400> 4
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Asn Leu Phe Pro Lys
   20 25 30
   Val Ala Pro Gln Ala Ile Ser Ser Val Glu Asn Ile Glu Gly Asn Gly
   35 40 45
   Gly Pro Gly Thr Ile Lys Asn Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 5
   <211> 159
   <212> PRT
   <213> Artificial Sequence
<220>
   <223> Description of Artificial Sequence: Modified Bet v 1 sequence, Thr77Ala modification
<400> 5
   Gly Val Phe Asn Tyr Glu Thr Glu Thr Thr Ser Val Ile Pro Ala Ala
   1               5                   10                  15
   Arg Leu Phe Lys Ala Phe Ile Leu Asp Gly Asp Asn Leu Phe Pro Lys
               20                  25                  30
   Val Ala Pro Gln Ala Ile Ser Ser Val Glu Asn Ile Glu Gly Asn Gly
           35                  40                  45
   Gly Pro Gly Thr Ile Lys Lys Ile Ser Phe Pro Glu Gly Leu Pro Phe
       50                  55                  60
   Lys Tyr Val Lys Asp Arg Val Asp Glu Val Asp His Ala Asn Phe Lys
   65                  70                  75                  80
   Tyr Asn Tyr Ser Val Ile Glu Gly Gly Pro Ile Gly Asp Thr Leu Glu
                   85                  90                  95
   Lys Ile Ser Asn Glu Ile Lys Ile Val Ala Thr Pro Asp Gly Gly Ser
               100                 105                 110
   Ile Leu Lys Ile Ser Asn Lys Tyr His Thr Lys Gly Asp His Glu Val
           115                 120                 125
   Lys Ala Glu Gln Val Lys Ala Ser Lys Glu Met Gly Glu Thr Leu Leu
       130                 135                 140
   Arg Ala Val Glu Ser Tyr Leu Leu Ala His Ser Asp Ala Tyr Asn
   145                 150                 155
<210> 6
   <211> 159
   <212> PRT
   <213> Artificial Sequence
<220>
   <223> Description of Artificial Sequence: Modified Bet v 1 sequence, Glu60Ser modification
<400> 6
   Gly Val Phe Asn Tyr Glu Thr Glu Thr Thr Ser Val Ile Pro Ala Ala
   1               5                   10                  15
   Arg Leu Phe Lys Ala Phe Ile Leu Asp Gly Asp Asn Leu Phe Pro Lys
               20                  25                  30
   Val Ala Pro Gln Ala Ile Ser Ser Val Glu Asn Ile Glu Gly Asn Gly
   35 40 45
   Gly Pro Gly Thr Ile Lys Lys Ile Ser Phe Pro Ser Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 7
   <211> 159
   <212> PRT
   <213> Artificial sequence
<220>
   <223> Description of Artificial Sequence: Modified Bet v 1 sequence, Asp25Gly modification
<400> 7
   Gly Val Phe Asn Tyr Glu Thr Glu Thr Thr Ser Val Ile Pro Ala Ala
   1 5 10 15
   Arg Leu Phe Lys Ala Phe Ile Leu Gly Gly Asp Asn Leu Phe Pro Lys
   20 25 30
   Val Ala Pro Gln Ala Ile Ser Ser Val Glu Asn Ile Glu Gly Asn Gly
   35 40 45
   Gly Pro Gly Thr Ile Lys Lys Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 8
   <211> 159
   <212> PRT
   <213> Artificial Sequence
<220>
   <223> Description of Artificial Sequence: Modified Bet v 1 sequence, Asn47Ser modification
<400> 8
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Asn Leu Phe Pro Lys
   20 25 30
   Val Ala Pro Gln Ala Ile Ser Ser Val Glu Asn Ile Glu Gly Ser Gly
   35 40 45
   Gly Pro Gly Thr Ile Lys Lys Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Pro Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 9
   <211> 159
   <212> PRT
   <213> Artificial Sequence
<220>
   <223> Description of Artificial Sequence: Modified Bet v 1 sequence, Pro108Gly modification
<400> 9
   Gly Val Phe Asn Tyr Glu Thr Glu Thr Thr Ser Val Ile Pro Ala Ala
   1 5 10 15
   Arg Leu Phe Lys Ala Phe Ile Leu Asp Gly Asp Asn Leu Phe Pro Lys
   20 25 30
   Val Ala Pro Gln Ala Ile Ser Ser Val Glu Asn Ile Glu Gly Asn Gly
   35 40 45
   Gly Pro Gly Thr Ile Lys Lys Ile Ser Phe Pro Glu Gly Leu Pro Phe
   50 55 60
   Lys Tyr Val Lys Asp Arg Val Asp Glu Val Asp His Thr Asn Phe Lys
   65 70 75 80
   Tyr Asn Tyr Ser Val Ile Glu Gly Gly Pro Ile Gly Asp Thr Leu Glu
   85 90 95
   Lys Ile Ser Asn Glu Ile Lys Ile Val Ala Thr Gly Asp Gly Gly Ser
   100 105 110
   Ile Leu Lys Ile Ser Asn Lys Tyr His Thr Lys Gly Asp His Glu Val
   115 120 125
   Lys Ala Glu Gln Val Lys Ala Ser Lys Glu Met Gly Glu Thr Leu Leu
   130 135 140
   Arg Ala Val Glu Ser Tyr Leu Leu Ala His Ser Asp Ala Tyr Asn
   145 150 155
<210> 10
   <211> 159
   <212> PRT
   <213> Artificial Sequence
<220>
   <223> Description of Artificial Sequence: Modified Bet v 1 sequence, Asn28Thr, Lys32Gln, Glu45Ser, Pro108Gly modification
<400> 10
   Gly Val Phe Asn Tyr Glu Thr Glu Thr Thr Ser Val Ile Pro Ala Ala
   1               5                   10                  15
   Arg Leu Phe Lys Ala Phe Ile Leu Asp Gly Asp Thr Leu Phe Pro Gln
               20                  25                  30
   Val Ala Pro Gln Ala Ile Ser Ser Val Glu Asn Ile Ser Gly Asn Gly
           35                  40                  45
   Gly Pro Gly Thr Ile Lys Lys Ile Ser Phe Pro Glu Gly Leu Pro Phe
       50                  55                  60
   Lys Tyr Val Lys Asp Arg Val Asp Glu Val Asp His Thr Asn Phe Lys
   65                  70                  75                  80
   Tyr Asn Tyr Ser Val Ile Glu Gly Gly Pro Ile Gly Asp Thr Leu Glu
                   85                  90                  95
   Lys Ile Ser Asn Glu Ile Lys Ile Val Ala Thr Gly Asp Gly Gly Ser
               100                 105                 110
   Ile Leu Lys Ile Ser Asn Lys Tyr His Thr Lys Gly Asp His Glu Val
           115                 120                 125
   Lys Ala Glu Gln Val Lys Ala Ser Lys Glu Met Gly Glu Thr Leu Leu
       130                 135                 140
   Arg Ala Val Glu Ser Tyr Leu Leu Ala His Ser Asp Ala Tyr Asn
   145                 150                 155
<210> 11
   <211> 204
   <212> PRT
   <213> Artificial Sequence
<220>
   <223> Description of Artificial Sequence: Modified Ves v 5 sequence, Lys72Ala modification
<400> 11
   Asn Asn Tyr Cys Lys Ile Lys Cys Leu Lys Gly Gly Val His Thr Ala
    1               5                  10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Ala Asn Met Lys Asn Leu Val Trp Asn
   65                  70                  75                      80
   Asp Glu Leu Ala Tyr Val Ala Gln Val Trp Ala Asn Gln Cys Gln Tyr
                   85                  90                  95
   Gly His Asp Thr Cys Arg Asp Val Ala Lys Tyr Gln Val Gly Gln Asn
               100                 105                 110
   Val Ala Leu Thr Gly Ser Thr Ala Ala Lys Tyr Asp Asp Pro Val Lys
           115                 120                 125
   Leu Val Lys Met Trp Glu Asp Glu Val Lys Asp Tyr Asn Pro Lys Lys
       130                 135                 140
   Lys Phe Ser Gly Asn Asp Phe Leu Lys Thr Gly His Tyr Thr Gln Met
   145                 150                 155                     160
   Val Trp Ala Asn Thr Lys Glu Val Gly Cys Gly Ser Ile Lys Tyr Ile
                   165                 170                 175
   Gln Glu Lys Trp His Lys His Tyr Leu Val Cys Asn Tyr Gly Pro Ser
               180                 185                 190
   Gly Asn Phe Lys Asn Glu Glu Leu Tyr Gln Thr Lys
           195                 200
<210> 12
   <211> 204
   <212> PRT
   <213> Artificial Sequence
<220>
   <223> Description of Artificial Sequence: Modified Ves v 5 sequence, Tyr96Ala modification
<400> 12
   Asn Asn Tyr Cys Lys Ile Lys Cys Leu Lys Gly Gly Val His Thr Ala
    1               5                  10                  15
   Cys Lys Tyr Gly Ser Leu Lys Pro Asn Cys Gly Asn Lys Val Val Val
               20                  25                  30
   Ser Tyr Gly Leu Thr Lys Gln Glu Lys Gln Asp Ile Leu Lys Glu His
           35                  40                  45
   Asn Asp Phe Arg Gln Lys Ile Ala Arg Gly Leu Glu Thr Arg Gly Asn
       50                  55                  60
   Pro Gly Pro Gln Pro Pro Ala Lys Asn Met Lys Asn Leu Val Trp Asn
   65                  70                  75                  80
   Asp Glu Leu Ala Tyr Val Ala Gln Val Trp Ala Asn Gln Cys Gln Ala
                   85                  90                  95
   Gly His Asp Thr Cys Arg Asp Val Ala Lys Tyr Gln Val Gly Gln Asn
               100                 105                 110
   Val Ala Leu Thr Gly Ser Thr Ala Ala Lys Tyr Asp Asp Pro Val Lys
           115                 120                 125
   Leu Val Lys Met Trp Glu Asp Glu Val Lys Asp Tyr Asn Pro Lys Lys
       130                 135                 140
   Lys Phe Ser Gly Asn Asp Phe Leu Lys Thr Gly His Tyr Thr Gln Met
   145                 150                 155                 160
   Val Trp Ala Asn Thr Lys Glu Val Gly Cys Gly Ser Ile Lys Tyr Ile
                   165                 170                 175
   Gln Glu Lys Trp His Lys His Tyr Leu Val Cys Asn Tyr Gly Pro Ser
               180                 185                 190
   Gly Asn Phe Lys Asn Glu Glu Leu Tyr Gln Thr Lys
           195                 200

## Description

### FIELD OF THE INVENTION

The present invention relates to novel recombinant allergens, which are non-naturally occurring mutants derived from naturally occurring allergens. Further, the invention relates to a method of preparing such recombinant allergens as well as to pharmaceutical compositions, including vaccines, comprising the recombinant allergens. In further embodiments, the present invention relates to methods of generating immune responses in a subject, vaccination or treatment of a subject as well as processes for preparing the compositions of the invention.

### BACKGROUND OF THE INVENTION

Genetically predisposed individuals become sensitised (allergic) to antigens originating from a variety of environmental sources, to the allergens of which the individuals are exposed. The allergic reaction occurs when a previously sensitised individual is re-exposed to the same or a homologous allergen. Allergic response range from hay fever, rhinoconductivitis, rhinitis and asthma to systemic anaphylaxis and death in response to e.g. bee or hornet sting or insect bite. The reaction is immediate and can be caused by a variety of atopic allergens such as compounds originating from grasses, trees, weeds, insects, food, drugs, chemicals and perfumes.

However, the responses do not occur when an individual is exposed to an allergen for the first time. The initial adoptive response takes time and does usually not cause any symptoms. But when antibodies and T cells capable of reacting with the allergen have been produced, any subsequent exposure may provoke symptoms. Thus, allergic responses demonstrate that the immune response itself can cause significant pathological states, which may be life threatening.

The antibodies involved in atopic allergy belong primarily to immunoglobulins of the IgE class. IgE binds to specific receptors on the surface of mast cells and basophils. Following complex formation of a specific allergen with IgE bound to mast cells, receptor cross-linking on the cell surface results in signalling through the receptors and the physiological response of the target cells. Degranulation results in the release of i.a. histamine, heparin, a chemotactic factor for eosinophilic leukocytes, leukotrienes C4, D4 and E4, which cause prolonged constriction of the bronchial smooth muscle cells. The resulting effects may be systemic or local in nature.

The antibody-mediated hypersensitivity reactions can be divided into four classes, namely type I, type II, type III and type IV. Type I allergic reactions is the classic immediate hypersensitivity reaction occurring within seconds or minutes following antigen exposure. These symptoms are mediated by allergen specific IgE.

Commonly, allergic reactions are observed as a response to protein allergens present e.g. in pollens, house dust mites, animal hair and dandruff, venoms, and food products.

In order to reduce or eliminate allergic reactions, carefully controlled and repeated administration of allergy vaccines is commonly used. Allergy vaccination is traditionally performed by parenteral, intranasal, or sublingual administration in increasing doses over a fairly long period of time, and results in desensitisation of the patient. The exact immunological mechanism is not known, but induced differences in the phenotype of allergen specific T cells is thought to be of particular importance.

### Antibody-binding epitopes (B-cell epitopes)

X-ray crystallographic analyses of F_{ab}-antigen complexes has increased the understanding of antibody-binding epitopes. According to this type of analysis antibody-binding epitopes can be defined as a section of the surface of the antigen comprising atoms from 15-25 amino acid residues, which are within a distance from the atoms of the antibody enabling direct interaction. The affinity of the antigen-antibody interaction can not be predicted from the enthalpy contributed by van der Waals interactions, hydrogen bonds or ionic bonds, alone. The entropy associated with the almost complete expulsion of water molecules from the interface represent an energy contribution similar in size. This means that perfect fit between the contours of the interacting molecules is a principal factor underlying antigen-antibody high affinity interactions.

### Allergy vaccination

The concept of vaccination is based on two fundamental characteristics of the immune system, namely specificity and memory. Vaccination will prime the immune system of the recipient, and upon repeated exposure to similar proteins the immune system will be in a position to respond more rigorously to the challenge of for example a microbial infection. Vaccines are mixtures of proteins intended to be used in vaccination for the purpose of generating such a protective immune response in the recipient. The protection will comprise only components present in the vaccine and homologous antigens.

Compared to other types of vaccination allergy vaccination is complicated by the existence of an ongoing immune response in allergic patients. This immune response is characterised by the presence of allergen specific IgE mediating the release of allergic symptoms upon exposure to allergens. Thus, allergy vaccination using allergens from natural sources has an inherent risk of side effects being in the utmost consequence life threatening to the patient.

Approaches to circumvent this problem may be divided in three categories. In practise measures from more than one category are often combined. First category of measures includes the administration of several small doses over prolonged time to reach a substantial accumulated dose. Second category of measures includes physical modification of the allergens by incorporation of the allergens into gel substances such as aluminium hydroxide. Aluminium hydroxide formulation has an adjuvant effect and a depot effect of slow allergen release reducing the tissue concentration of active allergen components. Third category of measures include chemical modification of the allergens for the purpose of reducing allergenicity, i.e. IgE binding.

The detailed mechanism behind successful allergy vaccination remains controversial. It is, however, agreed that T cells play a key role in the overall regulation of immune responses. According to current consensus the relation between two extremes of T cell phenotypes, Th1 and Th2, determine the allergic status of an individual. Upon stimulation with allergen Th1 cells secrete interleukines dominated by interferon-γ leading to protective immunity and the individual is healthy. Th2 cells on the other hand secrete predominantly interleukin 4 and 5 leading to IgE synthesis and eosinophilia and the individual is allergic. *In vitro* studies have indicated the possibility of altering the responses of allergen specific T cells by challenge with allergen derived peptides containing relevant T cell epitopes. Current approaches to new allergy vaccines are therefore largely based on addressing the T cells, the aim being to silence the T cells (anergy induction) or to shift the response from the Th2 phenotype to the Th1 phenotype.

In WO 97/30150 (ref. 1), a population of protein molecules is claimed, which protein molecules have a distribution of specific mutations in the amino acid sequence as compared to a parent protein. From the description, it appears that the invention is concerned with producing analogues which are modified as compared to the parent protein, but which are taken up, digested and presented to T cells in the same manner as the parent protein (naturally occurring allergens). Thereby, a modified T cell response is obtained. Libraries of modified proteins are prepared using a technique denoted PM (Parsimonious Mutagenesis).

In WO 92/02621 (ref. 2), recombinant DNA molecules are described, which molecules comprise a DNA coding for a polypeptide having at least one epitope of an allergen of trees of the order *Fagales*, the allergen being selected from *Aln g* 1, *Cor a* 1 and *Bet v* 1. The recombinant molecules described herein do all have an amino acid sequence or part of an amino acid sequence that corresponds to the sequence of a naturally occurring allergen.

WO 90/11293 (ref. 3) relates i.a. to isolated allergenic peptides of ragweed pollen and to modified ragweed pollen peptides. The peptides disclosed therein have an amino acid sequence corresponding either to the sequence of the naturally occurring allergen or to naturally occurring isoforms thereof.

### Chemical modification of allergens

Several approaches to chemical modification of allergens have been taken. Approaches of the early seventies include chemical coupling of allergens to polymers, and chemical cross-linking of allergens using formaldehyde, etc., producing the so-called 'allergoids'. The rationale behind these approaches was random destruction of IgE binding epitopes by attachment of the chemical ligand thereby reducing IgE-binding while retaining immunogenicity by the increased molecular weight of the complexes. Inherent disadvantages of 'allergoid' production are linked to difficulties in controlling the process of chemical cross-linking and difficulties in analysis and standardisation of the resulting high molecular weight complexes. 'Allergoids' are currently in clinical use and due to the random destruction of IgE binding epitopes higher doses can be administered as compared to conventional vaccines, but the safety and efficacy parameters are not improved over use of conventional vaccines.

More recent approaches to chemical modification of allergens aim at a total disruption of the tertiary structure of the allergen thus eliminating IgE binding assuming that the essential therapeutic target is the allergen specific T cell. Such vaccines contain allergen sequence derived synthetic peptides representing minimal T cells epitopes, longer peptides representing linked T cells epitopes, longer allergen sequence derived synthetic peptides representing regions of immunodominant T cell epitopes, or allergen molecules cut in two halves by recombinant technique. Another approach based on this rationale has been the proposal of the use of "low IgE binding" recombinant isoforms. In recent years it has become clear that natural allergens are heterogeneous containing isoallergens and variants having up to approximately 25% of their amino acids substituted. Some recombinant isoallergens have been found to be less efficient in IgE binding possibly due to irreversible denaturation and hence total disruption of tertiary structure.

### In vitro mutagenesis and allergy vaccination

Attempts to reduce allergenicity by *in vitro* site directed mutagenesis have been performed using several allergens including Der f 2 (Takai *et al*, ref. 4), Der p 2 (Smith *et al*, ref. 5), a 39 kDa *Dermatophagoides farinae* allergen (Aki *et al*, ref. 6), bee venom phospholipase A2 (Förster *et al,* ref. 7), Ara h 1 (Burks *et al,* ref. 8), Ara h 2 (Stanley *et al,* ref. 9), *Bet v* 1 (Ferreira *et al,* ref. 10 and 11), birch profilin (Wiedemann *et al,* ref. 12), and Ory s 1 (Alvarez *et al,* ref. 13).

The rationale behind these approaches, again, is addressing allergen specific T cells while at the same time reducing the risk of IgE mediated side effects by reduction or elimination of IgE binding by disruption of the tertiary structure of the recombinant mutant allergen. The rationale behind these approaches does not include the concept of dominant IgE binding epitopes and it does not include the concept of initiating a new protective immune response which also involves B-cells and antibody generation.

The article by Ferreira *et a*l (ref. 11) describes the use of site directed mutagenesis for the purpose of reducing IgE binding. Although the three-dimensional structure of *Bet v* 1 is mentioned in the article the authors do not use the structure for prediction of surface exposed amino acid residues for mutation, half of which have a low degree of solvent exposure. Rather they use a method developed for prediction of functional residues in proteins different from the concept of structure based identification of conserved surface areas described here. Although the authors do discuss conservation of α-carbon backbone tertiary structure this concept is not a part of the therapeutic strategy but merely included to assess *in vitro* IgE binding. Furthermore, the evidence presented is not adequate since normalisation of CD-spectra prevents the evaluation of denaturation of a proportion of the sample, which is a common problem. The therapeutic strategy described aim at inducing tolerance in allergen specific T cells and initiation of a new immune response is not mentioned.

The article by Wiedemann *et al*. (ref. 12) describes the use of site directed mutagenesis and peptide synthesis for the purpose of monoclonal antibody epitope characterisation. The authors have knowledge of the tertiary structure of the antigen and they use this knowledge to select a surface exposed amino acid for mutation. The algorithm used can be said to be opposite to the one described by the present inventors since an amino acid differing from homologous sequences is selected. The study demonstrates that substitution of a surface exposed amino acid has the capacity to modify the binding characteristics of a monoclonal antibody, which is not surprising considering common knowledge. The experiments described are not designed to assess modulation in the binding of polyclonal antibodies such as allergic patients' serum IgE. One of the experiments contained do apply serum IgE and although this experiment is not suitable for quantitative assessment, IgE binding does not seem to be affected by the mutations performed.

The article by Smith *et al*. (ref. 5) describes the use of site directed mutagenesis for the purpose of monoclonal antibody epitope mapping and reduction of IgE binding. The authors have no knowledge of the tertiary structure and make no attempt to assess the conservation of α-carbon backbone tertiary structure. The algorithm used does not ensure that amino acids selected for mutation are actually exposed to the molecular surface. Only one of the mutants described lead to a substantial reduction in IgE binding. This mutant is deficient in binding of all antibodies tested indicating that the tertiary structure is disrupted. The authors do not define a therapeutic strategy and initiation of a new immune response is not mentioned.

The article by Colombo *et al*. (ref. 14) describes the study of an IgE binding epitope by use of site directed mutagenesis and peptide synthesis. The authors use a three dimensional computer model structure based on the crystal structure of a homologous protein to illustrate the presence of the epitope on the molecular surface. The further presence of an epitope on a different allergen, showing primary structure homology is addressed using synthetic peptides representing the epitope. The therapeutic strategy is based on treatment using this synthetic peptide representing a monovalent IgE binding epitope. Conserved surface areas between homologous allergens as well as the therapeutic concept of initiating a new protective immune response are not mentioned.

The article by Spangfort *et al*. (ref. 15) describes the three-dimensional structure and conserved surface exposed patches of the major birch allergen. The article does not mention major IgE binding epitopes nor site directed mutagenesis, neither is therapeutic application addressed.

In none of the studies described above is IgE binding reduced by substitution of surface exposed amino acids while conserving α-carbon backbone tertiary structure. The rationale behind above-mentioned approaches does not include the concept of dominant IgE binding epitopes and it does not include the therapeutic concept of initiating a new protective immune response.

### BRIEF DESCRIPTION OF THE FIGURES

Figure 1 shows mutant-specific oligonucleotide primers used for *Bet v* 1 mutant number 1. Mutated nucleotides are underlined.
Figure 2 shows two generally applicable primers (denoted "all-sense" and "all non-sense"), which were synthesised and used for all mutants.
Figure 3 shows an overview of all *Bet v* 1 mutations.
Figure 4 shows the inhibition of the binding of biotinylated recombinant *Bet v* 1 to serum IgE from a pool of allergic patients by non-biotinylated *Bet v* 1 and by *Bet v* 1 Glu45Ser mutant.
Figure 5 shows the inhibition of the binding of biotinylated recombinant *Bet v* 1 to serum IgE from a pool of allergic patients by non-biotinylated *Bet v* 1 and by *Bet v* 1 mutant Asn28Thr+Lys32Gln.
Figure 6 shows the inhibition of the binding of biotinylated recombinant *Bet v* 1 to serum IgE from a pool of allergic patients by non-biotinylated *Bet v* 1 and by *Bet v* 1 Pro108Gly mutant.
Figure 7 shows the inhibition of the binding of biotinylated recombinant *Bet v* 1 to serum IgE from a pool of allergic patients by non-biotinylated Bet *v* 1 and by *Bet v* 1 Glu60Ser mutant.
Figure 8 shows the CD spectra of recombinant and Triple-patch mutant, recorded at close to equal concentrations.
Figure 9 shows the inhibition of the binding of biotinylated recombinant *Bet v* 1 to serum IgE from a pool of allergic patients by non-biotinylated *Bet v* 1 and by *Bet v* 1 Triple-patch mutant.
Figure 10 shows solvent accessibility of individually aligned antigen 5 residues and alignment of *Vespula* antigen 5 sequences (left panel). On the right panel of Figure 10 is shown the molecular surface of antigen 5 with conserved areas among *Vespula* antigen 5:s.
Figure 11 shows the sequence of the primer corresponding to the amino terminus of *Ves v* 5 derived from the sense strand. The sequence of the downstream primer is derived from the non-sense strand.
Figure 12 shows two generally,applicable primers (denoted "all sense" and "all non-sense", which were synthesised and used for all mutants.
Figure 13 shows an overview of all *Ves v* 5 mutations.
Figure 14 shows the inhibition of the binding of biotinylated recombinant *Ves v* 5 to serum IgE from a pool of allergic patients by non-biotinylated *Ves v* 5 and by *Ves v* 5 Lys72Ala mutant.

### OBJECT OF THE INVENTION

### Rationale behind the present invention

The current invention is based on a unique rationale. According to this rationale the mechanism of successful allergy vaccination is not an alteration of the ongoing Th2-type immune response, but rather a parallel initiation of a new Th1-type immune response involving tertiary epitope recognition by B-cells and antibody formation. This model is supported by the observation that levels of specific IgE are unaffected by successful vaccination treatment, and that successful treatment is often accompanied by a substantial rise in allergen specific IgG4. In addition, studies of nasal biopsies before and after allergen challenge do not show a reduction in T cells with the Th2-like phenotype, but rather an increase in Th1-like T cells are observed. When the vaccine (or pharmaceutical compositions) is administered through another route than the airways, it is hypothesised, that the new Th1-like immune response evolves in a location physically separated from the ongoing Th2 response thereby enabling the two response to exist in parallel.

Another important aspect of the rationale behind the current invention is the assertion of the existence of dominant IgE binding epitopes. It is proposed that these dominant IgE binding epitopes are constituted by tertiary structure dependent coherent surface areas large enough to accommodate antibody binding and conserved among isoallergens, variants, and/or homologous allergens from related species. The existence of cross-reactive IgE capable of binding similar epitopes on homologous allergens is supported by the clinical observation that allergic patients often react to several closely related species, e.g. alder, birch, and hazel, multiple grass species, or several species of the house dust mite genus Dermatophagoides. It is furthermore supported by laboratory experiments demonstrating IgE cross-reactivity between homologous allergens from related species and the capacity of one allergen to inhibit the binding of IgE to homologous allergens (Ipsen *et al.* 1992, ref. 16). It is well known that exposure and immune responses are related in a dose dependent fashion. Based on the combination of these observations it is hypothesised that conserved surface areas are exposed to the immune system in higher doses than non-conserved surface areas resulting in the generation of IgE antibodies with higher affinities, hence the term 'dominant IgE binding epitopes'.

According to this rationale it is essential that the allergen has an α-carbon backbone tertiary structure which essentially is the same as that of the natural allergen, thus ensuring conservation of the surface topology of areas surrounding conserved patches representing targets for mutagenesis aimed at reducing IgE binding. By fulfilling these criteria the allergen has the potential to be administered in relatively higher doses improving its efficacy in generating a protective immune response without compromising safety.

### SUMMARY OF THE INVENTION

The present invention relates to the introduction of artificial amino acid substitutions into defined critical positions while retaining the α-carbon backbone tertiary structure of the allergen.

The invention provides a recombinant allergen, which is a non-naturally occurring mutant derived from a naturally occurring allergen, wherein at least one surface-exposed, conserved amino acid residue of a B cell epitope is substituted by another residue which does not occur in the same position in the amino acid sequence of any known homologous protein within the taxonomic order from which said naturally occurring allergen originates, said mutant allergen having essentially the same α-carbon backbone tertiary structure as said naturally occurring allergen, and the specific IgE binding to the mutated allergen being reduced as compared to the binding to said naturally occurring allergen.

The invention provides a non-naturally occurring recombinant mutant allergen derived from a naturally occurring allergen, said naturally occurring allergen comprising at least one patch of amino acid residues, which are coherently connected over at least 400 Å2 of the surface of the three-dimensional structure of the allergen molecule, which are having a solvent accessibility of at least 20% and which are conserved as defined by being identical in more than 70 % of the sequences of homologues sequences within the same taxonomical order from which said naturally occurring allergen originates, said patch comprising at least one B cell epitope, wherein
1) said mutant comprising at least one substitution of a conserved amino acid residue located in said at least one patch and, said conserved amino acid residue having a solvent accessibility of at least 20 %, with an amino acid residue, which does not occur in the same position in the amino acid sequence of any known homologous protein within the taxonomic order from which said naturally occurring allergen originates,
2) said mutant allergen having essentially the same α-carbon backbone tertiary structure as said naturally occurring allergen, and
3) the specific IgE binding to the mutated allergen being reduced as compared to the binding to said naturally occurring allergen.

Such recombinant allergen is obtainable by
a) identifying amino acid residues in a naturally occurring allergen which are conserved with more than 70% identity in all known homologous proteins within the taxonomic order from which said naturally occurring allergen originates;
b) defining at least one patch of conserved amino acid residues being coherently connected over at least 400 A² of the surface of the three-dimensional of the allergen molecule as defined by having a solvent accessibility of at least 20%, said at least one patch comprising at least one B cell epitope; and
c) substituting at least one amino acid residue in said at least one patch by another amino acid being non-con-servative in the particular position while essentially preserving the overall α-carbon backbone tertiary structure of the allergen molecule.

Specific IgE binding to the mutated allergen is preferably reduced by at least 5%, preferably at least 10% in comparison to naturally-occurring isoallergens or similar recombinant proteins in an immune assay with sera from scource-specific IgE reactive allergic patients or pools thereof.

Recombinant allergens according to the invention may suitably be derived from inhalation allergens originating i.a. from trees, grasses, herbs, fungi, house dust mites, cockroaches and animal hair and dandruff. Important pollen allergens from trees, grasses and herbs are such originating from the taxonomic orders of *Fagales, Oleales* and *Pinales* including i.a. birch (*Betula*), alder (*Alnus*), hazel (*Corylus*), hornbeam (*Carpinus*) and olive (*Olea*), the order of Poales including i.a. grasses of the genera *Lolium, Phelum, Poa, Cynodon, Dactylis* and Secale, the orders of *Asterales* and *Urticales* including i.a. herbs of the genera *Ambrosia* and *Artemisia*. Important inhalation allergens from fungi are i.a. such originating from the genera *Alternaria* and *Cladosporium*. Other important inhalation allergens are those from house dust mites of the genus *Dermatophagoides,* those from cockroaches and those from mammals such as cat, dog and horse. Further, recombinant allergens according to the invention may be derived from venom allergens including such originating from stinging or biting insects such as those from the taxonomic order of *Hymenoptera* including bees (superfamily Apidae), wasps (superfamily Vespidea), and ants (superfamily Formicoidae).

Specific allergen components include e.g. *Bet v* 1 (*B. verrucosa,* birch), *Aln g* 1 (Alnus glutinosa, alder), *Cor a* 1 (*Corylus avelana*, hazel) and *Car b* 1 (*Carpinus betulus*, hornbeam) of the *Fagales* order. Others are *Cry j* 1 (*Pinales*), *Amb a* 1 and 2, , *Art v* 1 (*Asterales*) , *Par j* 1 (*Urticales*), *Ole e* 1 (*Oleales*), *Ave e* 1, *Cyn d* 1, *Dac g* 1, *Fes p* 1, *Hol 1* 1, *Lol p* 1 and 5, *Pas n* 1, *Phl p* 1 and 5, *Poa p* 1, 2 and 5, *Sec c* 1 and 5, and *Sor h* 1 (various grass pollens), *Alt* a 1 and *Cla h* 1 (fungi), *Der f* 1 and 2, *Der p* 1 and 2 (house dust mites, *D. farinae* and *D. pteronyssinus*, respectively), *Bla g* 1 and 2, *Per a* 1 (cockroaches, *Blatella germanica and Periplaneta americana*, respectively), *Fel d* 1 (cat), *Can f* 1 (dog), *Equ c* 1, 2 and 3 (horse), *Apis m* 1 and 2 (honeybee), *Ves g* 1, 2 and 5, *Pol a* 1, 2 and 5 (all wasps) and *Sol i* 1, 2, 3 and 4 (fire ant).

In one embodiment, the recombinant allergen is derived from *Bet v* 1. Examples of substitutions are Thr10Pro, Asp25Gly, (Asn28Thr + Lys32Gln), Glu45Ser, Asn47Ser, Lys55Asn, Thr77Ala, Pro108Gly and (Asn28Thr, Lys32Gln, Glu45Ser, Pro108Gly). As apparent, the recombinant allergens may have one or more substitutions.

In another embodiment, the recombinant allergen is derived from a venom allergen from the taxonomic order of Vespidae, Apidae and Formicoidae.

In a further embodiment, the recombinant allergen is derived from *Ves v* 5. Examples of substitutions are Lys72Ala and Tyr96Ala. As apparent, the recombinant allergens may have one or more substitutions.

The present invention also provides a method of preparing a recombinant allergen as defined herein, comprising
a) identifying amino acid residues in a naturally occurring allergen which are conserved with more than 70% identity in all known homologous proteins within the taxonomic order from which said naturally occurring allergen originates;
b) defining at least one patch of conserved amino acid residues being coherently connected over at least 400 Å² of the surface of the three-dimensional structure of the allergen molecule as defined by having a solvent accessibility of at least 20 %, said at least one patch comprising at least one B cell epitope, and
c) substituting at least one amino acid residue in said at least one patch by another amino acid being non-conservative in the particular position while essentially preserving the overall α-carbon backbone tertiary structure of the allergen molecule.

In this method the best results are obtained by ranking the amino acid residues of said at least one patch with respect to solvent accessibility and substituting one or more amino acids among the more solvent accessible ones.

Generally, in the method according to the invention the substitution of one or more amino acid residues in said B cell epitope or said at least one patch is carried out by site-directed mutagenesis.

Conservation of α-carbon backbone tertiary structure is best determined by obtaining identical structures by x-ray crystallography or NMR before and after mutagenesis. In absence of structural data describing the mutant indistinguishable CD-spectra or immunochemical data, e.g. antibody reactivity, may render conservation of α-carbon backbone tertiary structure probable, if compared to the data obtained by analysis of a structurally determined molecule.

Further, the present invention provides a pharmaceutical composition comprising a recombinant allergen as defined herein in combination with a pharmaceutically acceptable carrier and/or excipient, and optionally an adjuvant.

Such pharmaceutical composition may be in the form of a vaccine against allergic reactions elicited by a naturally occurring allergen in patients suffering from allergy.

In a further aspect, the present invention relates to a method of generating an immune response in a subject, which method comprises administering to the subject at least one recombinant allergen as defined herein, or a pharmaceutical composition comprising at least one recombinant allergen as defined herein.

The pharmaceutical composition of the invention can be prepared by a process comprising mixing at least one recombinant allergen as defined herein with pharmaceutically acceptable substances and/or excipients.

In a particular embodiment, the present invention concerns the vaccination or treatment of a subject, which vaccination of treatment comprises administering to the subject at least one recombinant allergens as defined herein or a pharmaceutical composition as defined herein.

The pharmaceutical compositions of the invention are obtainable by the process defined above.

In another embodiment, the recombinant allergen of the invention are suitable for use in a method for the treatment, prevention or alleviation of allergic reactions, such method comprising administering to a subject a recombinant allergen as defined herein or a pharmaceutical composition as defined herein.

### DETAILED DESCRIPTION OF THE INVENTION

### Criteria for substitution

For molecules for which the tertiary structure has been determined (e.g. by x-ray crystallography, or NMR electron microscopy), the mutant carrying the substituted amino acid(s) should preferably fulfil the following criteria:
1. The overall α-carbon backbone tertiary structure of the molecule should be conserved. Conserved is defined as an average root mean square deviation of the atomic coordinates comparing the structures below 2Å. This is important for two reasons: a) It is anticipated that the entire surface of the natural allergen constitutes an overlapping continuum of potential antibody-binding epitopes. The majority of the surface of the molecule is not affected by the substitution(s), and thus retain its antibody-binding properties, which is important for the generation of new protective antibody specificities being directed at epitopes present also on the natural allergen. b) Stability, both concerning shelf-life and upon injection into body fluids.
2. The amino acid(s) to be substituted should be located at the surface, and thus be accessible for antibody-binding. Amino acids located on the surface are defined as amino acids in the three-dimensional structure having a solvent (water) accessibility of at least 20%, suitably 20-80%, more suitably 30-80%. Solvent accessibility is defined as the area of the molecule accessible to a sphere with a radius comparable to a solvent (water, r = 1.4 Å) molecule.
3. The substituted amino acid(s) should be located in conserved patches larger than 400 Å². Conserved patches are defined as coherently connected areas of surface exposed conserved amino acid residues and backbone. Conserved amino acid residues are defined by sequence alignment of all known (deduced) amino acid sequences of homologues proteins within the taxonomical order. Amino acid positions having identical amino acid residues in more than 90% of the sequences are considered conserved. Conserved patches are expected to contain epitopes to which the IgE of the majority of patients is directed.
4. Within the conserved patches amino acids for mutagenesis should preferentially be selected among the most solvent (water) accessible ones located preferably near the centre of the conserved patch.

Preferentially, a polar amino acid residue is substituted by another polar residue, and a non-polar amino acid residue is substituted by another non-polar residue.

Preparation of vaccines is generally well-known in the art. Vaccines are typically prepared as injectables either as liquid solutions or suspensions. Such vaccine may also be emulsified or formulated so as to enable nasal administration. The immunogenic component in question (the recombinant allergen as defined herein) may suitably be mixed with excipients which are pharmaceutically acceptable and further compatible with the active ingredient. Examples of suitable excipients are water, saline, dextrose, glycerol, ethanol and the like as well as combinations thereof. The vaccine may additionally contain other substances such as wetting agents, emulsifying agents, buffering agents or adjuvants enhancing the effectiveness of the vaccine.

Vaccines are most frequently administered parenterally by subcutaneous or intramuscular injection. Formulations which are suitable for administration by another route include oral formulations and suppositories. Vaccines for oral administration may suitably be formulated with excipients normally employed for such formulations, e.g. pharmaceutical grades of mannitol, lactose, starch, magnesium stearate, sodium saccharine, cellulose, magnesium carbonate and the like. The composition can be formulated as solutions, suspensions, emulsions, tablets, pills, capsules, sustained release formulations, aerosols, powders, or granulates.

The vaccines are administered in a way so as to be compatible with the dosage formulation and in such amount as will be therapeutically effective and immunogenic. The quantity of active component contained within the vaccine depends on the subject to be treated, i.a. the capability of the subject's immune system to respond to the treatment, the route of administration and the age and weight of the subject. Suitable dosage ranges can vary within the range from about 0.0001 pg to 1000 µg.

As mentioned above, an increased effect may be obtained by adding adjuvants to the formulation. Examples of such adjuvants are aluminum hydroxide and phosphate (alum) as a 0.05 to 0.1 percent solution in phosphate buffered saline, synthetic polymers of sugars used as 0.25 percent solution. Mixture with bacterial cells such as *C. parvum*, endotoxins or lipopolysaccharide components of gram-negative bacteria, emulsion in physiologically acceptable oil vehicles such as mannide monoaleate (Aracel A) or emulsion with 20 percent solution of a perfluorocarbon (e.g. Fluosol-DA) used as a block substitute may also be employed. Other adjuvants such as Freund's complete and incomplete adjuvants as well as QuilA and RIBI may also be used.

Most often, multiple administrations of the vaccine will be necessary to ensure an effect. Frequently, the vaccine is administered as an initial administration followed by subsequent inoculations or other administrations. The number of vaccinations will typically be in the range of from 1 to 50, usually not exceeding 35 vaccinations. Vaccination will normally be performed from biweekly to bimonthly for a period of 3 months to 5 years. This is contemplated to give desired level of prophylactic or therapeutic effect.

The recombinant allergen may be used as a pharmaceutical preparation, which is suitable for providing a certain protection against allergic responses during the period of the year where symptoms occur (prophylaxis). Usually, the treatment will have to be repeated every year to maintain the protective effect. Preparations formulated for nasal application are particular suited for this purpose.

The present invention is further illustrated by the following non-limiting examples.

### EXAMPLES

### EXAMPLE 1

### Identification of common epitopes within Fagales pollen allergens

The major birch pollen allergen *Bet v* 1 shows about 90% amino acid sequence identity with major allergens from pollens of taxonomically related trees, i.e *Fagales* (for instance hazel and hornbeam) and birch pollen allergic patients often show clinical symptoms of allergic cross-reactivity towards these *Bet v* 1 homologous proteins.

*Bet v* 1 also shows about 50-60% sequence identity with allergic proteins present in certain fruits (for instance apple and cherry) and vegetables (for instance celery and carrot) and there are clinical evidence for allergic cross-reactivity between *Bet v* 1 and these food related proteins.

In addition, *Bet v* 1 shares significant sequence identity (20-40%) with a group of plant proteins called pathogenesis-related proteins (PR-10), however there are no reports of allergic cross-reactivity towards these PR-10 proteins.

Molecular modelling suggests that the structures of *Fagales* and food allergens and PR-10 proteins are close to be identical with the *Bet v* 1 structure.

The structural basis for allergic *Bet v* 1 cross-reactivity was reported in (Gajhede et al 1996, ref. 17) where three patches on the molecular surface of *Bet v* 1 could be identified to be common for the known major tree pollen allergens. Thus, any IgE recognising these patches on *Bet v* 1 would be able to cross-react and bind to other *Fagales* major pollen allergens and give rise to allergic symptoms. The identification of these common patches was performed after alignment of all known amino acid sequences of the major tree pollen allergens in combination with an analysis of the molecular surface of *Bet v* 1 revealed by the α-carbon backbone tertiary structure reported in ref. 17. In addition, the patches were defined to have a certain minimum size (>400 Å²) based on the area covered by an antibody upon binding.

### Selection of amino acid residues for site-directed mutagenesis

Amino acid residues for site-directed mutagenesis were selected among residues present in *Bet v* 1 specific areas and the common patches since modifications of these is expected to affect the binding of serum IgE from the majority of patients showing clinical tree pollen allergic cross-reactivity.

The relative orientation and percentage of solvent-exposure of each amino acid residue within respective patch was calculated based on their atomic coordinates. Residues having a low degree of solvent exposure (<20%) were not regarded relevant for mutagenesis due to the possible disruption of the structure or lack of antibody interaction. The remaining residues were ranked according to their degree of solvent-exposure.

### Sequence alignment

Sequences homologous to the query sequence (*Bet v* 1 No. 2801, WHO IUIS Nomenclature Subcommittee on Allergens) were derived from GenBank and EMBL sequence databases by a BLAST search (Altschul et al., ref. 18). All sequences with BLAST reported probabilities less than 0.1 were taken into consideration and one list were constructed containing a non-redundant list of homologous sequences. These were aligned by CLUSTAL W (Higgins et al., ref. 19) and the percentage identity were calculated for each position in the sequence considering the complete list or taxonomically related species only. A total of 122 sequences were homologous to *Bet v 1 No. 2801* of which 57 sequences originates from taxonomically related species.

### Cloning of the gene encoding Bet v 1

RNA was prepared from *Betula verrucosa* pollen (Allergon, Sweden) by phenol extraction and LiCl precipitation. Oligo(dT)-cellulose affinity chromatography was performed batch-wise in Eppendorph tubes, and double-stranded cDNA was synthesised using a commercially available kit. (Amersham). DNA encoding *Bet v* 1 was amplified by PCR and cloned. In brief, PCR was performed using cDNA as template, and primers designed to match the sequence of the cDNA in positions corresponding to the amino terminus of Bet *v* 1 and the 3'-untranslated region, respectively. The primers were extended in the 5'-ends to accommodate restriction sites (*Nco*I and *Hind*III) for directional cloning into pKK233-2.

### Subcloning into pMAL-c

The gene encoding *Bet v* 1 was subsequently subcloned into the maltose binding protein fusion vector pMAL-c (New England Biolabs). The gene was amplified by PCR and subcloned in frame with *malE* to generate maltose binding protein (MBP)-*Bet v* 1 protein fusion operons in which MBP and *Bet v* 1 were separated by a factor Xₐ protease clevage site positioned to restore the authentic aminoterminal sequence of *Bet v* 1 upon cleavage, as described in ref. 15. In brief, PCR was performed using pKK233-3 with *Bet v 1* inserted as template and primers corresponding to the amino- and carboxyterminus of the protein, respectively. The promoter proximal primer was extended in the 5'-end to accommodate 4 codons encoding an in frame factor Xₐ protease cleavage site. Both primers were furthermore extended in the 5'-ends to accommodate restriction sites (*Kpn*I) for cloning. The *Bet v* 1 encoding genes were subcloned using 20 cycles of PCR to reduce the frequency of PCR artefacts.

### In vitro mutagenesis

*In vitro* mutagenesis was performed by PCR using recombinant pMAL-c with *Bet v 1* inserted as template. Each mutant *Bet v* 1 gene was generated by 3 PCR reactions using 4 primers.

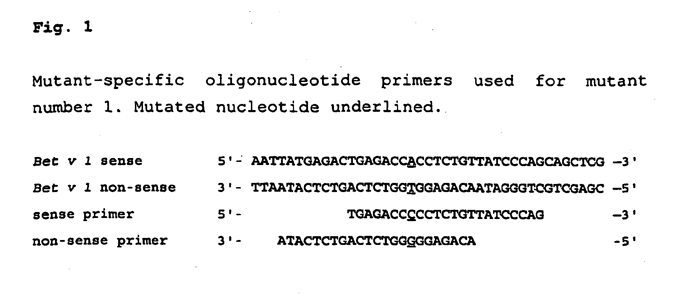

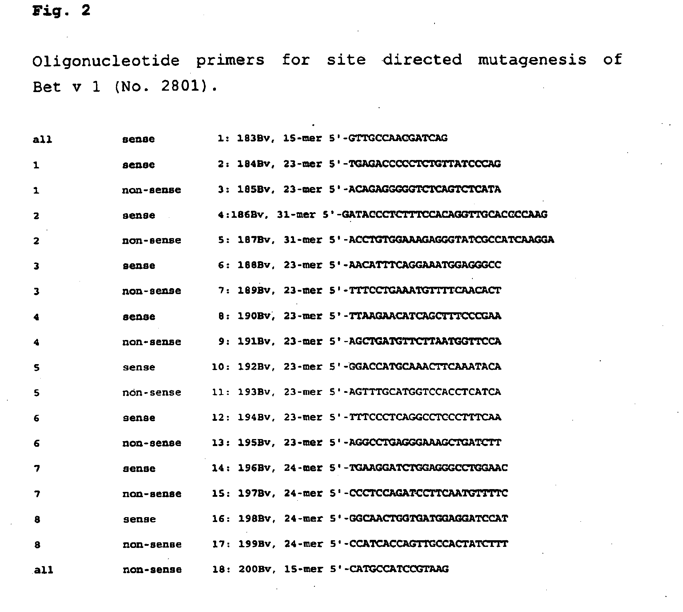

Two mutation-specific oligonucleotide primers were synthesised accommodating each mutation, one for each DNA strand, see Figs. 1 and 2, Using the mutated nucleotide(s) as starting point both primers were extended 7 nucleotides in the 5'-end and 15 nucleotides in the 3'-end. The extending nucleotides were identical in sequence to the Bet *v* 1 gene in the actual region.

Two generally applicable primers (denoted "all-sense" and "all non-sense" in Figure 2) were furthermore synthesised and used for all mutants. These primers were 15 nucleotides in length and correspond in sequence to regions of the pMAL-c vector approximately 1 kilobase upstream and downstream from the *Bet v* 1. The sequence of the upstream primer is derived from the sense strand and the sequence of the downstream primer is derived from the non-sense strand, see Fig. 2.

Two independent PCR reactions were performed essentially according to standard procedures (Saiki et al 1988, ref. 20) with the exception that only 20 temperature cycles were performed in order to reduce the frequency of PER artefacts. Each PCR reaction used pMAL-c with *Bet v* 1 inserted as template and one mutation-specific and one generally applicable primer in meaningful combinations.

Introduction of the four amino acid substitutions (Asn28Thr, Lys32Gln, Glu45Ser, Pro108Gly) in the Triple-patch mutant were performed like described above in a step by step process. First the Glu45Ser mutation then the Pro108Gly mutation and last the Asn28Thr, Lys32Gln mutations were introduced using pMAL-c with inserted *Bet v 1 No. 2801, Bet v 1 (Glu45Ser) , Bet v 1 (Glu45Ser, Pro108Gly)* as templates, respectively.

The PCR products were purified by agarose gel electrophoresis and electro-elution followed by ethanol precipitation. A third PCR reaction was performed using the combined PCR products from the first two PCR reactions as template and both generally applicable primers. Again, 20 cycles of standard PCR were used. The PCR product was purified by agarose gel electrophoresis and electro-elution followed by ethanol precipitation, cut with restriction enzymes (*Bsi*WI/*Eco*RI), and ligated directionally into pMAL-c with Bet *v 1* inserted restricted with the same enzymes.

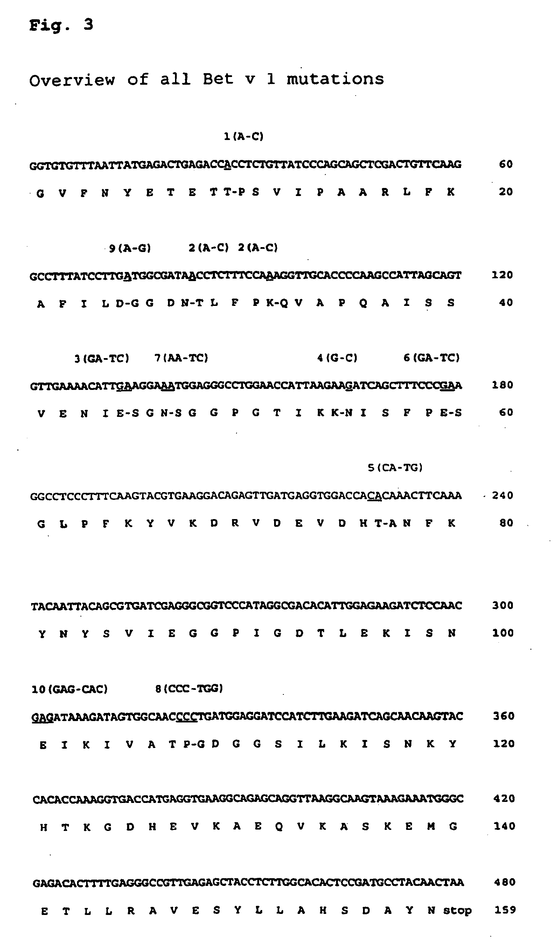

Figure 3 shows an overview of all 9 *Bet v* 1 mutations, which are as follows

Thr10Pro, Asp25Gly, Asn28Thr + Lys32Gln, Glu45Ser, Asn47Ser, Lys55Asn, Glu60Ser (non-patch), Thr77Ala and Pro108Gly. An additional four mutant with four mutations was also prepared (Asn28Thr, Lys32Gln, Glu45Ser, Pro108Gly). Of these, five were selected for further testing: Asn28Thr + Lys32Gln, Glu45Ser, Glu60ser, Pro108Gly and the Triple-patch mutant Asn28Thr, Lys32Gln, Glu45Ser, Pro108Gly.

### Nucleotide sequencing

Determination of the nucleotide sequence of the *Bet v* 1 encoding gene was performed before and after subcloning, and following *in vitro* mutagenesis, respectively.

Plasmid DNA's from 10 ml of bacterial culture grown to saturation overnight in LB medium supplemented with 0.1 g/l ampicillin were purified on Qiagen-tip 20 columns and sequenced using the Sequenase version 2.0 DNA sequencing kit (USB) following the recommendations of the suppliers.

### Expression and purification of recombinant Bet v 1 and mutants

Recombinant *Bet v* 1 (*Bet v* 1 No. 2801 and mutants) were over-expressed in *Escherichia coli* DH 5a fused to maltose-binding protein and purified as described in ref. 15. Briefly, recombinant *E. coli* cells were grown at 37°C to an optical density of 1.0 at 436 nm, whereupon expression of the Bet *v* 1 fusion protein was induced by addition of IPTG. Cells were harvested by centrifugation 3 hours post-induction, re-suspended in lysis buffer and broken by sonication. After sonication and additional centrifugation, recombinant fusion protein was isolated by amylose affinity chromatography and subsequently cleaved by incubation with Factor Xa (ref. 15). After F Xa cleavage, recombinant *Bet v* 1 was isolated by gelfiltration and if found necessary, subjected to another round of amylose affinity chromatography in order to remove trace amounts of maltose-binding protein.

Purified recombinant *Bet v* 1 was concentrated by ultrafiltration to about 5 mg/ml and stored at 4°C. The final yields of the purified recombinant *Bet v* 1 preparations were between 2-5 mg per litre *E. coli* cell culture.

The purified recombinant *Bet v* 1 preparations appeared as single bands after silver-stained SDS-polyacrylamide electrophoresis with an apparent molecular weight of 17.5 kDa. N-terminal sequencing showed the expected sequences as derived from the cDNA nucleotide sequences and quantitative amino acid analysis showed the expected amino acid compositions.

We have previously shown (ref. 15) that recombinant *Bet v* 1 No. 2801 is immunochemically indistinguishable from naturally occurring *Bet v* 1.

### Immunoelectrophoresis using rabbit polyclonal antibodies

The seven mutant *Bet v* 1 were produced as recombinant *Bet v* 1 proteins and purified as described above and tested for their reactivity towards polyclonal rabbit antibodies raised against *Bet v* 1 isolated from birch pollen. When analysed by immunoelectrophoresis (rocket-line immunoelectrophoresis) under native conditions, the rabbit antibodies were able to precipitate all mutants, indicating that the mutants had conserved α-carbon backbone tertiary structure.

These results suggested that non-naturally occurring substitutions introduced on the molecular surface of *Bet v* 1 can reduce a polyclonal antibody response raised against naturally occurring *Bet v* 1 without distortion of the overall α-carbon backbone tertiary allergen structure. In order to analyse the effect on human polyclonal IgE-response, the mutants Glu45Ser, Pro108Gly, Asn28Thr+Lys32Gln and Glu60Ser were selected for further analysis.

### Bet v 1 Glu45Ser mutant

Glutamic acid in position 45 show a high degree of solvent-exposure (40%) and is located in a molecular surface patch common for *Fagales* allergens (patch I). A serine residue was found to occupy position 45 in some of the *Bet v* 1 homologous PR-10 proteins arguing for that glutamic acid can be replaced by serine without distortion of the α-carbon backbone tertiary structure. In addition, as none of the known *Fagales* allergen sequences have serine in position 45, the substitution of glutamic acid with serine gives rise to a non-naturally occurring *Bet v* 1 molecule.

### T cell proliferation assay using recombinant Glu45Ser Bet v 1 mutant

The analysis was carried out as described in Spangfort *et al* 1996a. It was found that recombinant *Bet v* 1 Glu45Ser mutant was able to induce proliferation in T cell lines from three different birch pollen allergic patients with stimulation indices similar to recombinant and naturally occurring.

### Crystallisation and structural determination of recombinant Glu45Ser Bet v 1

Crystals of recombinant Glu45Ser *Bet v* 1 were grown by vapour diffusion at 25°C, essentially as described in (Spangfort et al 1996b, ref. 21). Glu45Ser *Bet v* 1, at a concentration of 5 mg/ml, was mixed with an equal volume of 2.0 M ammonium sulphate, 0.1 M sodium citrate, 1% (v/v) dioxane, pH 6.0 and equilibrated against 100x volume of 2.0 M ammonium sulfate, 0.1 M sodium citrate, 1% (v/v) dioxane, pH 6.0. After 24 hours of equilibration, crystal growth was induced by applying the seeding technique described in ref. 21, using crystals of recombinant wild-type *Bet v* 1 as a source of seeds.

After about 2 months, crystals were harvested and analysed using X-rays generated from a Rigaku rotating anode as described in ref. 21 and the structure was solved using molecular replacement.

### Structure of Bet v 1 Glu45Ser mutant

The structural effect of the mutation was addressed by growing three-dimensional *Bet v* 1 Glu45Ser protein crystals diffracting to 3.0 Å resolution when analysed by X-rays generated from a rotating anode. The substitution of glutamic acid to serine in position 45 was verified by the *Bet v* 1 Glu45Ser structure electron density map which also showed that the overall α-carbon backbone tertiary structure is preserved.

### IgE-binding properties of Bet v 1 Glu45Ser mutant

The IgE-binding properties of *Bet v* 1 Glu45Ser mutant was compared with recombinant *Bet v* 1 in a fluid-phase IgE-inhibition assay using a pool of serum IgE derived from birch allergic patients.

Recombinant *Bet v* 1 no. 2801 was biotinylated at a molar ratio of 1:5 (*Bet v* 1 no. 2801:biotin). The inhibition assay was performed as follows: a serum sample (25 µl) was incubated with solid phase anti IgE, washed, re-suspended and further incubated with a mixture of biotinylated *Bet v* 1 no. 2801 (3.4 nM) and a given mutant (0-28.6 nM). The amount of biotinylated *Bet v* 1 no. 2801 bound to the solid phase was estimated from the measured RLU after incubation with acridinium ester labelled streptavidin. The degree of inhibition was calculated as the ratio between the RLU's obtained using buffer and mutant as inhibitor.

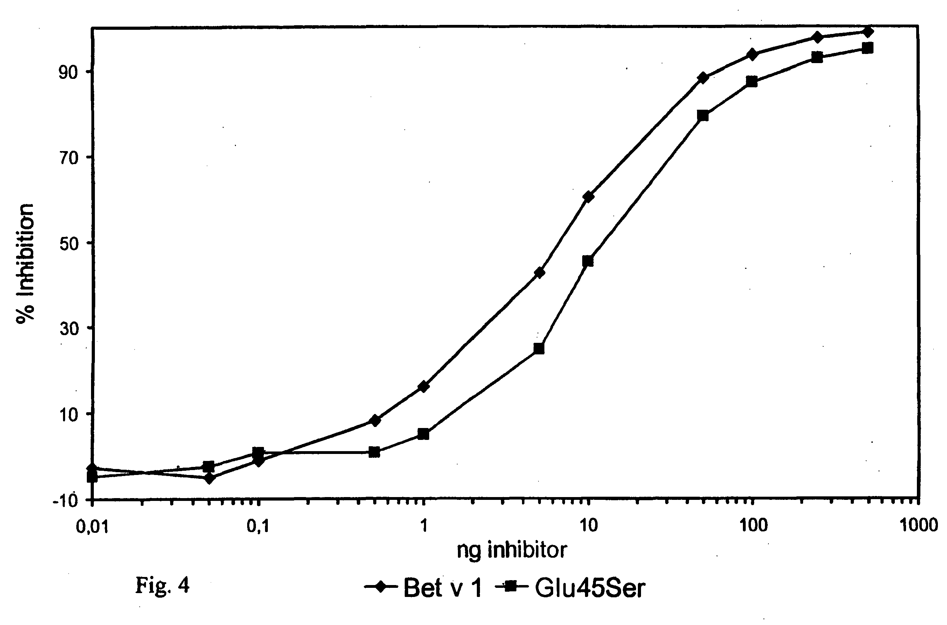

Figure 4 shows the inhibition of the binding of biotinylated recombinant *Bet v* 1 to serum IgE from a pool of allergic patients by non-biotinylated *Bet v* 1 and by *Bet v* 1 Glu45Ser mutant.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v 1* Glu45Ser mutant is about 12 ng. This show that the point mutation introduced in *Bet v* 1 Glu45Ser mutant lowers the affinity for specific serum IgE by a factor of about 2. The maximum level of inhibition reached by the *Bet v 1* Glu45Ser mutant is clearly lower compared to recombinant *Bet v* 1. This may indicate that after the Glu45Ser substitution, some of the specific IgE present in the serum pool are unable to recognise the *Bet v* 1 Glu45Ser mutant.

### Bet v 1 mutant Asn28Thr+Lys32Gln

Aspartate and lysine in positions 28 and 32, respectively show a high degree of solvent-exposure (35% and 50%, respectively) and are located in a molecular surface patch common for *Fagales* allergens (patch II). In the structure, aspartate 28 and lysine 32 are located close to each other on the molecular surface and most likely interact via hydrogen bonds. A threonine and a gluatamate residue were found to occupy positions 28 and 32, respectively in some of the *Bet v* 1 homologous PR-10 proteins arguing for that aspartate and lysine can be replaced with threonine and glutamate, respectively without distortion of the α-carbon backbone tertiary structure. In addition, as none of the naturally occurring isoallergen sequences have threonine and glutamate in positions 28 and 32, respectively, the substitutions gives rise to a non-naturally occurring *Bet v* 1 molecule.

### IgE-binding properties of Bet v 1 mutant Asn28Thr+Lys32Gln

The IgE-binding properties of mutant Asn28Thr+Lys32Gln was compared with recombinant *Bet v* 1 in a fluid-phase IgE-inhibition assay using the pool of serum IgE derived from birch allergic patients described above.

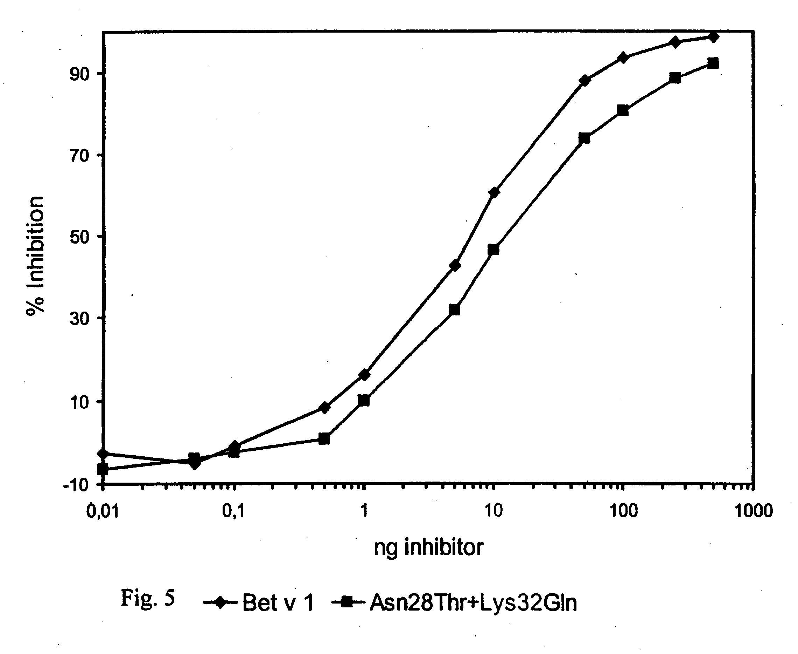

Figure 5 shows the inhibition of the binding of biotinylated recombinant *Bet v* 1 to serum IgE from a pool of allergic patients by non-biotinylated *Bet v* 1 and by *Bet v* 1 mutant Asn28Thr+Lys32Gln.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v 1* mutant Asn28Thr+Lys32Gln is about 12 ng. This show that the point mutations introduced in *Bet v* 1 mutant Asn28Thr+Lys32Gln lowers the affinity for specific serum IgE by a factor of about 2.

The maximum level of inhibition reached by the *Bet v 1* mutant Asn28Thr+Lys32Gln mutant is clearly lower compared to recombinant *Bet v* 1. This may indicate that after the Asn28Thr+Lys32Gln substitutions, some of the specific IgE present in the serum pool are unable to recognise the *Bet v 1* mutant Asn28Thr+Lys32Gln.

### Bet v 1 mutant Pro108Gly

Proline in position 108 show a high degree of solvent-exposure (60%) and is located in a molecular surface patch common for *Fagales* allergens (patch III). A glycine residue was found to occupy position 108 in some of the *Bet v* 1 homologous PR-10 proteins arguing for that proline can be replaced with glycine without distortion of the α-carbon backbone tertiary structure. In addition, as none of the naturally occurring isoallergen sequences have glycine in position 108, the substitution of proline with glycine gives rise to a non-naturally occurring *Bet v* 1 molecule.

### IgE-binding properties of Bet v 1 Pro108Gly mutant

The IgE-binding properties of *Bet v* 1 Pro108Gly mutant was compared with recombinant *Bet v* 1 in a fluid-phase IgE-inhibition assay using the pool of serum IgE derived from birch allergic patients described above.

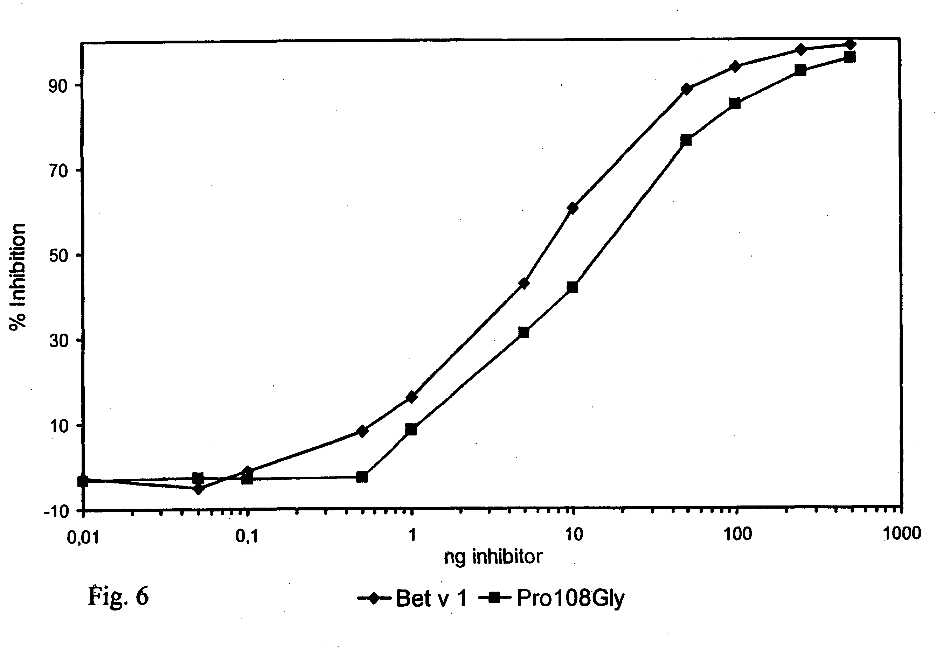

Figure 6 shows the inhibition of the binding of biotinylated recombinant *Bet v* 1 to serum IgE from a pool of allergic patients by non-biotinylated *Bet v* 1 and by *Bet v* 1 Pro108Gly mutant.

There is a clear difference in the amount of respective recombinant proteins necessary to reach 50% inhibition of the binding to serum IgE present in the serum pool. Recombinant *Bet v* 1 reaches 50% inhibition at about 6.5 ng whereas the corresponding concentration for *Bet v* 1 Pro108Gly is 15 ng. This show that the single point mutation introduced in *Bet v* 1 Pro108Gly lowers the affinity for specific serum IgE by a factor of about 2.

The maximum level of inhibition reached by the *Bet v* 1 Pro108Gly mutant is somewhat lower compared to recombinant *Bet v* 1. This may indicate that after the Pro108Gly substitution, some of the specific IgE present in the serum pool are unable to recognise the *Bet v* 1 Pro108Gly mutant.

### Bet v 1 mutant Glu60Ser (non-patch mutant)

Glutamic acid in position 60 show a high degree of solvent-exposure (60%) however, it is not located in a molecular surface patch common for *Fagales* allergens. A serine residue was found to occupy position 60 in some of the *Bet v* 1 homologous PR-10 proteins arguing for that glutamic acid can be replaced with serine without distortion of the α-carbon backbone tertiary structure. In addition, as none of the naturally occurring isoallergen sequences have serine in position 60, the substitution of glutamic acid with serine gives rise to a non-naturally occurring *Bet v* 1 molecule.

### IgE-binding properties of Bet v 1 Glu60Ser mutant

The IgE-binding properties of *Bet v* 1 Glu60Ser mutant was compared with recombinant *Bet v* 1 in a fluid-phase IgE-inhibition assay using the pool of serum IgE derived from birch allergic patients described above.

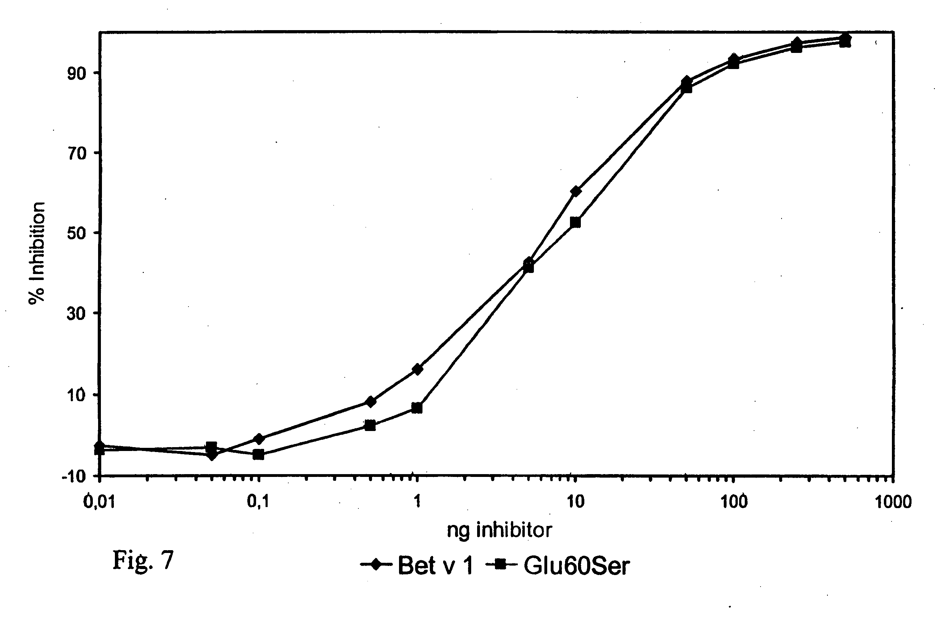

Figure 7 shows the inhibition of the binding of biotinylated recombinant *Bet v* 1 to serum IgE from a pool of allergic patients by non-biotinylated *Bet v* 1 and by *Bet v* 1 Glu60Ser mutant. In contrast to the Glu45Ser, Pro108Gly and Asn28Thr+Lys32Gln mutants, the substitution glutamic acid 60 to serine, does not shown any significant effect on the IgE-binding properties of. This indicates that substitutions outside the defined *Fagales* common patches only have a marginal effect on the binding of specific serum IgE supporting the concept that conserved allergen molecular surface areas harbours dominant IgE-binding epitopes.

### Bet v 1 Triple-patch mutant

In the Triple-patch mutant, the point mutations (Glu45Ser, Asn28Thr+Lys32Gln and Pro108Gly) introduced in the three different common *Fagales* patches, described above, were simultaneously introduced in creating an artificial mutant carrying four amino acid substitutions.

### Structural analysis of Bet v 1 Triple-patch mutant

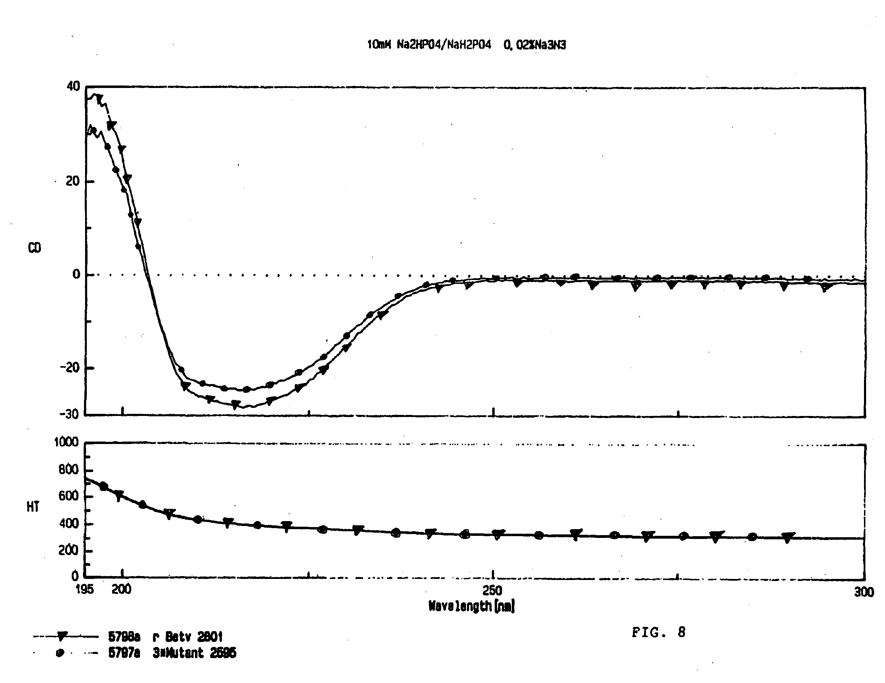

The structural integrity of the purified Triple-patch mutant was analysed by circular dichroism (CD) spectroscopy. Figure 8 shows the CD spectra of recombinant and Triple-patch mutant, recorded at close to equal concentrations. The overlap in peak amplitudes and positions in the CD spectra from the two recombinant proteins shows that the two preparations contain equal amounts of secondary structures strongly suggesting that the α-carbon backbone tertiary structure is not affected by the introduced amino acid substitutions.

### IgE-binding properties of Bet v 1 Triple-patch mutant

The IgE-binding properties of *Bet v* 1 Triple-patch mutant was compared with recombinant *Bet v* 1 in a fluid-phase IgE-inhibition assay using the pool of serum IgE derived from birch allergic patients described above.

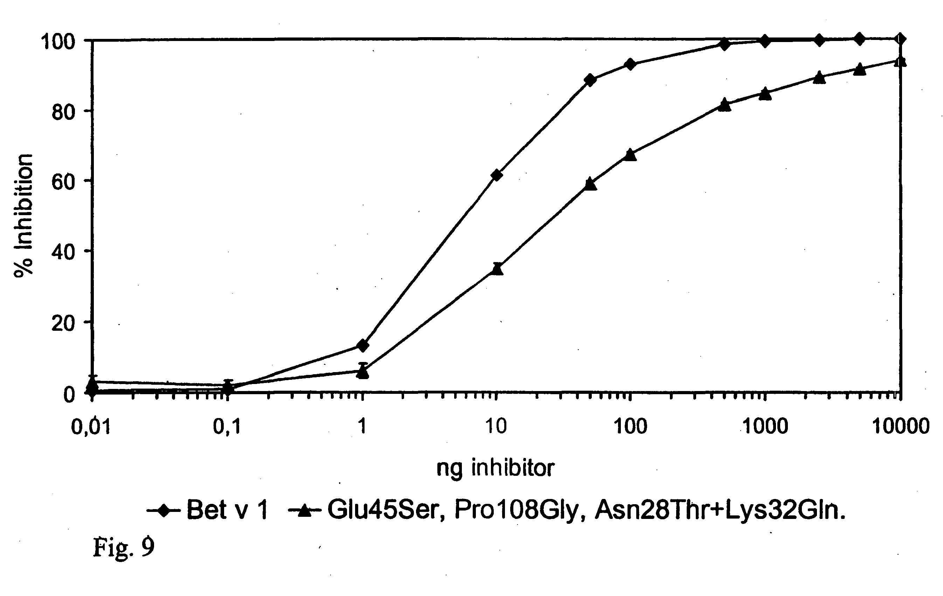

Figure 9 shows the inhibition of the binding of biotinylated recombinant *Bet v* 1 to serum IgE from a pool of allergic patients by non-biotinylated *Bet v* 1 and by *Bet v* 1 Triple-patch mutant. In contrast to the single mutants described above, the inhibition curve of the Triple-patch mutant is no longer parallel relative to recombinant. This shows that the substitutions introduced in the Triple-patch mutant has changed the IgE-binding properties and epitope profile compared to recombinant. The lack of parallellity makes it difficult to quantify the decrease of the Triple-patch mutant affinity for specific serum IgE.

Recombinant *Bet v* 1 reaches 50% inhibition at about 6 ng whereas the corresponding concentration for *Bet v* 1 Triple-patch mutant is 30 ng, i.e a decrease in affinity by a factor 5. However, in order to reach 80% inhibition the corresponding values are 20 ng and 400 ng, respectively, i.e a decrease by a factor 20.

### T cell proliferation assay using recombinant Bet v 1 Triple-patch mutant

The analysis was carried out as described in ref. 15. It was found that recombinant *Bet v* 1 Triple-patch mutant was able to induce proliferation in T cell lines from three different birch pollen allergic patients with stimulation indices similar to recombinant and naturally occurring. This suggests that the Triple-patch mutant can initiate the cellular immune response necessary for antibody production.

### EXAMPLE 2

### Identification of common epitopes within Vespula vulgaris venom major allergen antigen 5

Antigen 5 is one of the three vespid venom proteins, which are known allergens in man. The vespids include hornets, yellow-jacket and wasps. The other two known allergens of vespid venoms are phospholipase A₁ and hyaluronidase. Antigen 5 from *Vespula vulgaris* (*Ves v* 5) has been cloned and expressed as recombinant protein in the yeast system (Monsalve et al. 1999, ref. 22). The three-dimensional crystal structure of recombinant *Ves v* 5 has recently been determined at 1.8 Å resolution (in preparation). The main features of the structure consist of four β-strands and four α-helices arranged in three stacked layers giving rise to a "α-β-α sandwich". The sequence identity between Antigen 5 homologous allergens from different *Vespula* species is about 90% suggesting presence of conserved molecular surface areas and B cell epitopes.

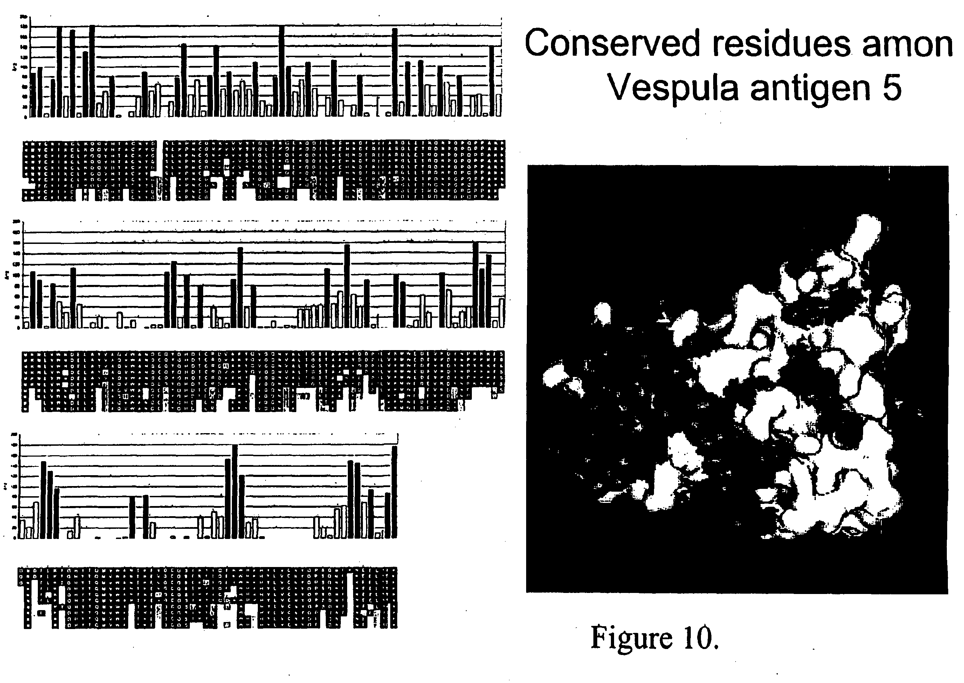

The presence and identification of common patches was performed after alignment of all known amino acid sequences, as previously described for tree pollen allergens, of the *Vespula* antigen 5 allergens in combination with an analysis of the molecular surface of Antigen 5 revealed by the three-dimensional structure of Ves v 5. Figure 10 shows solvent accessibility of individually aligned antigen 5 residues and alignment of *Vespula* antigen 5 sequences (left panel). On the right panel of figure 10 is shown the molecular surface of antigen 5 with conserved areas among *Vespula* antigen 5:s coloured.

### Selection of amino acid residues for site-directed mutagenesis

Amino acid residues for site-directed mutagenesis were selected among residues present the patches common for *Vespula* since modifications of these is expected to affect the binding of serum IgE from the majority of patients showing clinical *Vespula* allergic cross-reactivity.

The relative orientation and percentage of solvent-exposure of each amino acid residue within respective patch was calculated based on their atomic coordinates. Residues having a low degree of solvent exposure were not regarded suitable for mutagenesis due to the possible disruption of the structure or lack of antibody interaction. The remaining residues were ranked according to their degree of solvent-exposure.

### Cloning of the gene encoding Ves v 5

Total RNA was isolated from venom acid glands of *Vespula vulgaris* vespids as described in (Fang *et al*. 1988, ref. 23).

First-strand cDNA synthesis, PCR amplification and cloning of the *Ves v* 5 gene was performed as described in (Lu et al. 1993, ref. 24)

### Subcloning into pPICZαA

The gene encoding *Ves v* 5 was subsequently sub-cloned into the pPICZαA vector (Invitrogen) for secreted expression of *Ves v* 5 in *Pichia pastoris.* The gene was amplified by PCR and sub-cloned in frame with the coding sequence for the α-factor secretion signal of *Saccharomyces cerevisiae.* In this construct the α-factor is cleaved off, *in vivo,* by the *Pichia pastoris* Kex2 protease system during secretion of the protein.

In brief PCR was performed using *Ves v* 5 as template and primers corresponding to the amino- and carboxyterminus of the protein, respectively. The primers were extended in the 5'-end to accommodate restriction sites for cloning, EcoRI and XbaI, respectively. Nucleotides encoding the Kex2 cleavage site was in this construct positioned 18 nucleotides upstream to the amino terminus of the protein, resulting in the expression of Ves *v* 5 with six additional amino acids, Glu-Ala-Glu-Ala-Glu-Phe, at the amino terminus.

### Insertion of pPICZαA-Ves v 5 into P. pastoris

The pPICZαA vectors with the *Ves v* 5 gene inserted was linearised by Sac I restriction and inserted into the *AOX1* locus on the *Pichia pastoris* genome. Insertion was performed by homologous recombination on *Pichia pastoris* KM71 cells following the recommendations of Invitrogen.

### In vitro mutagenesis

*In vitro* mutagenesis was performed by PCR using recombinant pPICZαA with *Ves v* 5 inserted as template. Each mutant *Ves v 5* gene was generated by 3 PCR reactions using 4 primers.

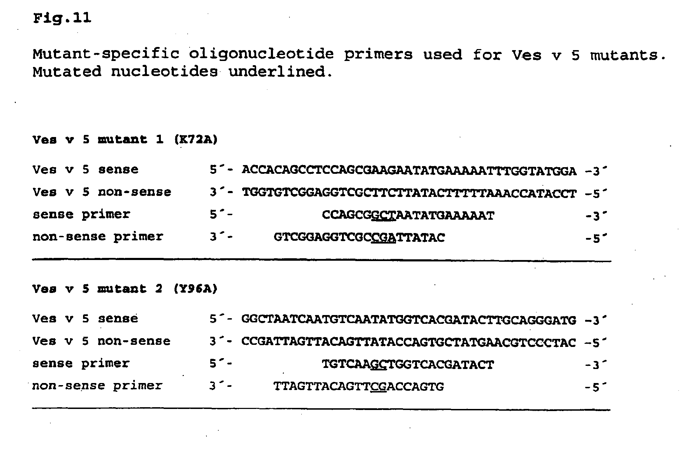

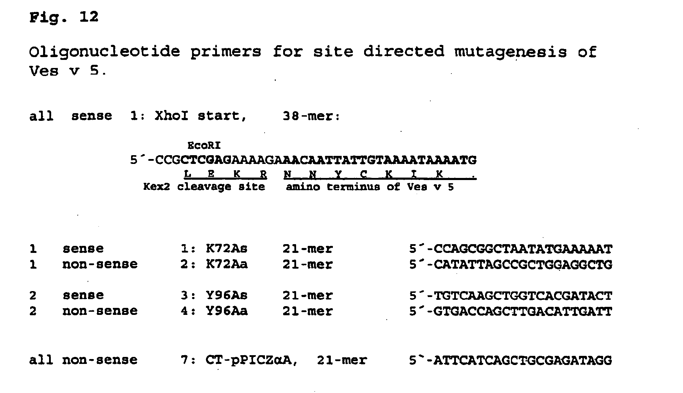

Two mutation-specific oligonucleotide primers were synthesised accommodating each mutation, one for each DNA strand, see Figures 11 and 12. Using the mutated nucleotide(s) as starting point both primers were extended 6-7 nucleotides in the 5'-end and 12-13 nucleotides in the 3'-end. The extending nucleotides were identical in sequence to the *Ves v* 5 gene in the actual region.

Two generally applicable primers (denoted "all sense" and "all non-sense" in Figure 12) were furthermore synthesised and used for all mutants. To insure expression of *Ves v* 5 mutants with authentic amino terminus, one primer corresponding to the amino terminus of the protein was extended in the 5'-end with a Xho I site. Upon insertion of the *Ves v* 5 mutant genes into the pPICZαA vector, the Kex2 protease cleavage site was regenerated directly upstream to the amino terminus of Ves v 5. The second primer was corresponding in sequence to a region of the pPICZαA vector positioned approximately 300 bp downstream from the *Ves v* 5 gene. The sequence of the primer corresponding to the amino terminus of *Ves v* 5 is derived from the sense strand and the sequence of the downstream primer is derived from the non-sense strand, see Figure 11.

Two independent PCR reactions were performed essentially according to standard procedures (Saiki *et al* 1988) with the exception that only 20 temperature cycles were performed in order to reduce the frequency of PCR artefacts. Each PCR reaction used pPICZαA with *Ves v 5* inserted as template and one mutation-specific and one generally applicable primer in meaningful combinations.

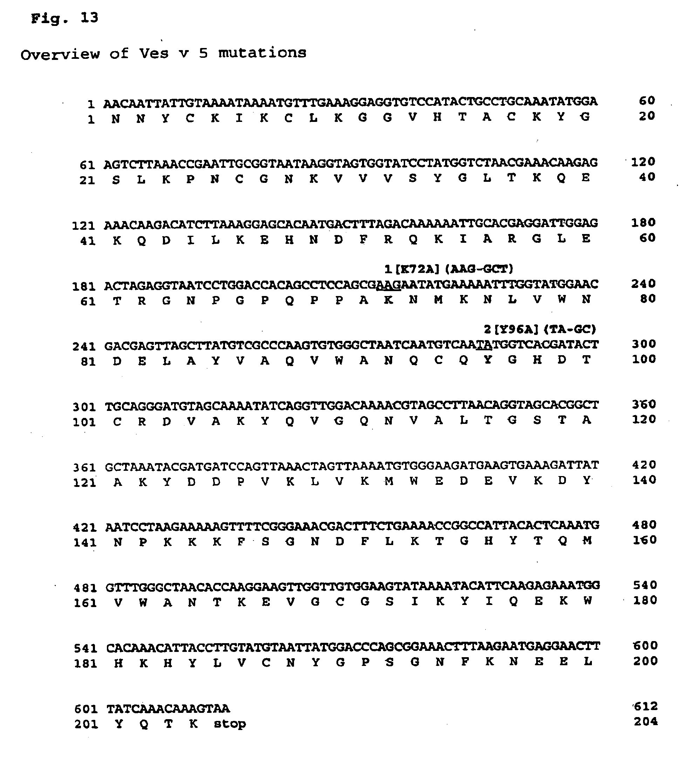

The PCR products were purified by using "Concert, Rapid PCR Purification System" (Life Technologies). A third PCR reaction was performed using the combined PCR products from the first two PCR reactions as template and both generally applicable primers. Again, 20 cycles of standard PCR were used. The PCR product was purified with the "Concert, Rapid PCR Purification System" (Life Technologies), cut with restriction enzymes (*XhoI*/*XbaI*), and ligated directionally into pPICZαA vector restricted with the same enzymes. Figure 13 shows an overview of all Ves v 5 mutations.

### Insertion of pPICZαA-Ves v 5 mutants into P. pastoris

The pPICZαA vectors with the *Ves v* 5 mutant genes inserted were linearised by Sac I restriction and inserted into the *AOX1* locus on the *Pichia pastoris* genome. Insertions were performed by homologous recombination on *Pichia pastoris* KM71 cells following the recommendations of Invitrogen.

### Nucleotide sequencing

Determination of the nucleotide sequence of the Ves v 5 encoding gene was performed before and after subcloning, and following in vitro mutagenesis, respectively.

Plasmid DNA's from 10 ml of bacterial culture grown to saturation overnight in LB medium supplemented with 0.1 g/l ampicillin were purified on Qiagen-tip 20 columns and sequenced using the Sequenase version 2.0 DNA sequencing kit (USB) following the recommendations of the suppliers.

### Expression and purification of recombinant Ves v 5

Recombinant yeast cells of *Pichia pastoris* strain KM71 were grown in 500 ml bottles containing 100 ml of pH 6.0 phosphate buffer containing yeast nitrogen base, biotin, glycerol and histidine at 30°C with orbital shaking at 225 rpm until A₅₀₀ nm of 4-6. Cells were collected by centrifugation and re-suspended in 10 ml of similar buffered medium containing methanol in place of glycerol. Incubation was continued at 30°C for 7 days with daily addition of 0.05 ml methanol.

Cells were harvested by centrifugation and the collected culture fluid was concentrated by ultrafiltration. After dialysis against 50 mM ammonium acetate buffer, pH 4.6, the sample was applied to a FPLC (Pharmacia) SE-53 cation exchange column equilibrated in the same buffer. The column was eluated with a 0-1.0 M NaCl, 50 mM ammonium acetate linear gradient. The recombinant Ves v 5 peak eluting at about 0.4 M NaCl was collected and dialysed against 0.02 N acetic acid. After concentration to about 10 mg/ml, the purified Ves v 5 was stored at 4°C.

### Crystallisation of recombinant Ves v 5

Crystals of *Ves v* 5 was grown by the vapour diffusion technique at 25°C. For crystallisation, 5 µl of 5 mg/ml Ves v 5 was mixed with 5 µl of 18% PEG 6000, 0.1 M sodium citrate, pH 6.0 and equilibrated against 1 ml of 18% PEG 6000, 0.1 M sodium citrate, pH 6.0.

X-ray diffraction data was collected at 100K from native *Ves v 5* crystals and after incorporation of heavy-atom derivatives and used to solve the three-dimensional structure of *Ves v* 5, see Figure 10 (manuscript in preparation).

### Immunoelectrophoresis using rabbit polyclonal antibodies

The two *Ves v* 5 mutants were produced as recombinant *Ves v* 5 proteins and tested for their reactivity towards polyclonal rabbit antibodies raised against recombinant *Ves v* 5. When analysed by rocket immunoelectrophoresis under native conditions, the rabbit antibodies were able to precipitate recombinant *Ves v* 5 as well as both mutants, indicating that the mutants have conserved α-carbon backbone tertiary structure.

### Inhibition of specific serum IgE

The IgE-binding properties of *Ves v* 5 mutants were compared to recombinant *Ves v* 5 in a fluid-phase IgE-inhibition assay using a pool of serum IgE derived from vespid venom allergic patients.

The inhibition assay was performed as described above using biotinylated recombinant *Ves v* 5 instead of *Bet v* 1.

### Ves v 5 Lys72Ala mutant

Lysine in position 72 show a high degree of solvent-exposure (70%) and is located in a molecular surface patch common for *Vespula* antigen 5. The relative orientation and high degree of solvent exposure argued for that lysine 72 can be replaced by an alanine residue without distortion of the α-carbon backbone tertiary structure. In addition, as none of the naturally occurring isoallergen sequences have alanine in position 72, the substitution of lysine with alanine gives rise to a non-naturally occurring *Ves v* 5 molecule.

### IgE-binding properties of Ves v 5 Lys72Ala mutant

The IgE-binding properties of *Ves v* 5 Lys72Ala mutant was compared with recombinant *Ves v* 5 in a fluid-phase IgE-inhibition assay using the pool of serum IgE derived from birch allergic patients described above.

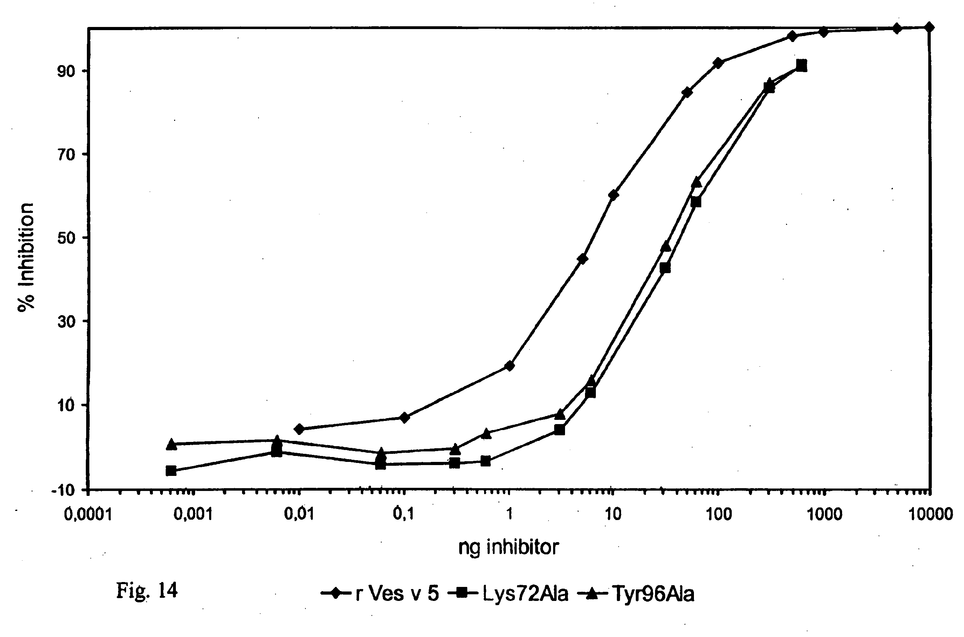

Figure 14 shows the inhibition of the binding of biotinylated recombinant *Ves v* 5 to serum IgE from a pool of allergic patients by non-biotinylated *Ves v* 5 and by *Ves v 5* Lys72Ala mutant.

There is a clear difference in the amount of respective recombinant proteins necessary to reach 50% inhibition of the binding to serum IgE present in the serum pool. Recombinant *Ves v* 5 reaches 50% inhibition at about 6 ng whereas the corresponding concentration for *Ves v* 5 Lys72Ala mutant is 40 ng. This show that the single point mutation introduced in *Ves v* 5 Lys72Ala mutant lowers the affinity for specific serum IgE by a factor of about 6. The maximum level of inhibition reached by the *Ves v* 5 Lys72Ala mutant significantly lower compared to recombinant *Ves v* 5. This may indicate that after the Lys72Ala substitution, some of the specific IgE present in the serum pool are unable to recognise the *Ves v* 5 Lys72Ala mutant.

### Ves v 5 Tyr96Ala mutant

Tyrosine in position 96 show a high degree of solvent-exposure (65%) and is located in a molecular surface patch common for *Vespula* antigen 5. The relative orientation an high degree of solvent exposure argued for that tyrosine 96 can be replaced by an alanine residue without distortion of the three-dimensional structure. In addition, as none of the naturally occurring isoallergen sequences have alanine in position 96, the substitution of tyrosine with alanine gives rise to a non-naturally occurring *Ves v* 5 molecule.

### IgE-binding properties of Ves v 5 Tyr96Ala mutant

The IgE-binding properties of *Ves v* 5 Tyr96Ala mutant was compared with recombinant *Ves v* 5 in a fluid-phase IgE-inhibition assay using the pool of serum IgE derived from birch allergic patients described above.

Figure 14 shows the inhibition of the binding of biotinylated recombinant *Ves v* 5 to serum IgE from a pool of allergic patients by non-biotinylated *Ves v* 5 and by *Ves v* 5 Tyr96Ala mutant.

There is a clear difference in the amount of respective recombinant proteins necessary to reach 50% inhibition of the binding to serum IgE present in the serum pool. Recombinant *Ves v* 5 reaches 50% inhibition at about 6 ng whereas the corresponding concentration for *Ves v 5* Tyr96Ala mutant is 40 ng.

This show that the single point mutation introduced in *Ves v* 5 Tyr96Ala mutant lowers the affinity for specific serum IgE by a factor of about 6.

The maximum level of inhibition reached by the *Ves v* 5 Tyr96Ala mutant significantly lower compared to recombinant *Ves v* 5. This may indicate that after the Tyr96Ala substitution, some of the specific IgE present in the serum pool are unable to recognise the *Ves v* 5 Tyr96Ala mutant.

### REFERENCES

1. WO 97/30150 (Pangenetics B.V., Molecules for the induction of immunological tolerance)
2. WO 92/02621 (Biomay Biotechnik Produktions- und Handelsgesellschaft mbH, Allergens of Alder pollen and applications thereof)
3. WO 90/11293 (Immunologic Pharmaceutical Corporation, The University of North Carolina at Chapel Hill, Allergenic proteins from ragweed and uses thereof)
4. Takai T, Yokota T, Yasue M, Nishiyama C, Yuuki T, Mori A, Okudaira H, Okumura Y: "Engineering of the major house dust mite allergen Der f 2 for allergen-specific immunotherapy". Nat Biotechnol 15, 754-758 (1997).
5. Smith AM, Chapman MD: "Localization of antigenic sites on Der p 2 using oligonucleotide-directed mutagenesis targeted to predicted surface residues". Clin Exp Allergy 27, 593-599 (1997).
6. Aki T, Ono K, Hidaka Y, Shimonishi Y, Jyo T, Wada T, Yamashita M, Shigeta S, Murooka Y, Oka S: "Structure of IgE epitopes on a new 39-kD allergen molecule from the house dust mite, Dermatophagoides farinae". Int Arch Allergy Immunol 103, 357-364 (1994).
7. Förster E, Dudler T, Gmachl M, Aberer W, Urbanek R, Suter M: "Natural and recombinant enzymatically active or inactive bee venom phospholipase A2 has the same potency to release histamine from basophils in patients with Hymenoptera allergy". J Allergy Clin Immunol 95, 1229-1235 (1995).
8. Burks AW, Shin D, Cockrell G, Stanley JS, Helm RM, Bannon GA: "Mapping and mutational analysis of the IgE-binding epitopes on Ara h 1, a legume vicilin protein and a major allergen in peanut hypersensitivity". Eur J Biochem 245, 334-339 (1997).
9. Stanley JS, King N, Burks AW, Huang SK, Sampson H, Cockrell G, Helm RM, West CM, Bannon GA: "Identification and mutational analysis of the immunodominant IgE binding epitopes of the major peanut allergen Ara h 2". Arch Biochem Biophys 342, 244-253 (1997).
10. Ferreira F, Rohlfs A, Hoffmann-Sommergruber K, Schenk S, Ebner C, Briza P, Jilek A,. Kraft D, Breitenbach M, Scheiner O: "Modulation of IgE-binding properties of tree pollen allergens by site-directed mutagenesis". Adv Exp Med Biol 409, 127-135 (1996).
11. Ferreira F, Ebner C, Kramer B, Casari G, Briza P, Kungl AJ, Grimm R, Jah-Schmid B, Breiteneder H, Kraft D, Breitenbach M, Rheinberger H-J, Scheiner O, "Modulation of IgE reactivity of allergens by site-directed mutagenesis: Potential use of hypeallergenic variants for immunotherapy", FASEB Journal for Experimental Biology Vol. 12, No. 2, February 1998, 231-242 (1998).
12. Wiedemann P, Giehl K, Almo SC, Fedorov AA, Girvin M, Steinberger P, Rüdiger M, Ortner M, Sippl M, Dolecek C, Kraft D, Jockusch B, Valenta R: "Molecular and structural analysis of a continuous birch profilin epitope defined by a monoclonal antibody". J Biol Chem 271, 29915-29921 (1996).
13. Alvarez AM, Fukuhara E, Nakase M, Adachi T, Aoki N, Nakamura R, Matsuda T: "Four rice seed cDNA clones belonging to the alpha-amylase/trypsin inhibitor gene family encode potential rice allergens". Biosci Biotechnol Biochem 59, 1304-1308 (1995).
14. Colombo P, Kennedy D, Ramsdale T, Costa MA, Djro G, Izzo V, Salvadori S, Guerrini R, Cocchiara R, Mirisola MG, Wood S, Geraci D, Journal of Immunology Vol. 160, No. 6, 15 March 1998, 2780-2875.
15. Spangfort MD, Ipsen H, Sparholt SH, Aasmul-Olsen S, Larsen MR, Mørtz E, Roepstorff P, Larsen JN: "Characterization of Purified Recombinant Bet v 1 with Authentic N-terminus, Cloned in Fusion with Maltose-Binding Protein". Prot Exp Purification 8, 365-373 (1996a).
16. Ipsen H, Wihl J-A, Petersen BN, Løwenstein H: "Specificity mapping of patients IgE response towards the tree pollen major allergens Aln g I, Bet v I and Cor a I." Clin. Exp. Allergy 22, 391-9, (1992)
17. Gajhede M, Osmark P, Poulsen FM, Ipsen H, Larsen JN, Joost van Neerven RJ, Schou C, Løwnstein H, and Spangfort MD: "X-ray and NMR structure of Bet v 1, the origin of birch pollen allergy". Nature structural biology 3, 1040-1045 (1996).
18. Altschul SF, Gish W, Miller W, Myers EW, and Lipman DJ: "Basic local alignment search tool". J. Mol. Biol. 215, 403-410 (1990).
19. Higgins D, Thompson J, Gibson T, Thompson JD, Higgins DG, and Gibson TJ: "CLUSTAL W: improving the sensitivity of progressive multiple sequence alignment through sequence weighting, position-specific gap penalties and weight matrix choice". Nucleic Acids Res. 22, 4673-4680 (1994).
20. Saiki RK, Gelfand DH, Stoffel S, Scharf SJ, Higuchi R, Horn GT, Mullis KB, Erlich HA: "Primer-directed enzymatic amplification of DNA with a thermostable DNA polymerase". Science 239, 487-491 (1988).
21. Spangfort MD, Larsen JN, Gajhede M: "Crystallization and Preliminary X-ray Investigation at 2.0 A Resolution of Bet v 1, a Birch Pollen Protein Causing IgE-Mediated Allergy". PROTEINS, Struc Func Genet 26, 358-360 (1996b).
22. Monsalve RI, Lu G, and King TP: "Recombinant venom allergen, antigen 5 of yellowjacket (Vespula vulgaris) and paper wasp (Polistes annularis) by expression in bacteria or yeast" (1999) Submitted.
23. Fang KSF, Vitale M, Fehlner P and King TP: "cDNA cloning and primary structure of a white-face hornet venom allergen, antigen 5". Proc. Natl. Acad. Sci. USA 85, 895 (1988).
24. Lu G, Villalba M, Coscia MR, Hoffman DR and King TP: "Sequence Analysis and Antigenic Cross-reactivity of a Venom Allergen, Antigen 5, from Hornets, Wasps, and Yellow Jackets". Journal of Immunology 150, 2823-2830 (1993).

## Claims

1. A non-naturally occurring recombinant mutant allergen derived from a naturally occurring allergen, said naturally occurring allergen comprising at least one patch of amino acid residues, which are coherently connected over at least 400 Å2 of the surface of the three-dimensional structure of the allergen molecule, which are having a solvent accessibility of at least 20% and which are conserved as defined by being identical in more than 70 % of the sequences of homologues sequences within the same taxonomical order from which said naturally occurring allergen originates, said patch comprising at least one B cell epitope, wherein
1) said mutant comprising at least one substitution of a conserved amino acid residue located in said at least one patch and, said conserved amino acid residue having a solvent accessibility of at least 20 %, with an amino acid residue, which does not occur in the same position in the amino acid sequence of any known homologous protein within the taxonomic order from which said naturally occurring allergen originates,
2) said mutant allergen having essentially the same α-carbon backbone tertiary structure as said naturally occurring allergen, and
3) the specific IgE binding to the mutated allergen being reduced as compared to the binding to said naturally occurring allergen.

2. Recombinant allergen according to claim 1, wherein the naturally occurring allergen is an inhalation allergen.

3. Recombinant allergen according to claim 2, wherein the naturally occurring allergen is a pollen allergen.

4. Recombinant allergen according to claim 3, wherein the naturally occurring allergen is a pollen allergen originating from the taxonomic order of Fagales, Oleales or Pinales.

5. Recombinant allergen according to claim 4, wherein the naturally occurring allergen is Bet v 1.

6. Recombinant allergen according to claim 5, wherein the substitution(s) is (are) Asp25Gly, (Asn28Thr + Lys32Gln), Glu45Ser, Asn47Ser, Lys55Asn, Thr77Ala, Pro108G1y or (Asn28Thr, Lys32Gln, Glu45Ser, Pro108Gly).

7. Recombinant allergen according to claim 3, wherein the naturally occurring allergen is a pollen allergen originating from the taxonomic order of Poales.

8. Recombinant allergen according to claim 3, wherein the naturally occurring allergen is a pollen allergen originating from the taxonomic order of Asterales or Urticales.

9. Recombinant allergen according to claim 1 or 2, wherein the naturally occurring allergen is a house dust mite allergen.

10. Recombinant allergen according to claim 9, wherein the naturally occurring allergen is a mite allergen originating from the taxonomic genus Dermatophagoides.

11. Recombinant allergen according to claim 1 or 2, wherein the naturally occurring allergen is a cockroach allergen.

12. Recombinant allergen according to claim 1 or 2, wherein the naturally occurring allergen is an animal allergen.

13. Recombinant allergen according to claim 12, wherein the naturally occurring allergen is an animal allergen originating from cat, dog or horse.

14. Recombinant allergen according to claim 1, wherein the naturally occurring allergen is a venom allergen.

15. Recombinant allergen according to claim 14, wherein the naturally occurring allergen is a venom allergen originating from the taxonomic order of Hymenoptera.

16. Recombinant allergen according to claim 15, wherein the naturally occurring allergen is a venom allergen originating from the taxonomic order of Vespidae, Apidae and Formicoidae.

17. Recombinant allergen according to claim 16, wherein the naturally occurring allergen is Ves v 5.

18. Recombinant allergen according to claim 17, wherein the substitution is Lys72Ala or Tyr96Ala.

19. Recombinant allergen according to any on of claims 1-18, wherein the specific IgE binding to the mutated allergen is reduced by at least 5%, preferably at least 100.

20. Recombinant allergen according to any on of claims 1-19, wherein when comparing the α-carbon backbone tertiary structures of the mutant and the naturally occurring allergen molecules, the average root mean square deviation of the atomic coordinates is below 2Å.

21. Recombinant allergen according to any on of claims 1-20, wherein said at least one patch comprises atoms of 15-25 amino acid residues.

22. Recombinant allergen according to any one of claims 1-21, wherein the amino acid residues of said at least one patch are ranked with respect to solvent accessibility, and one or more amino acids among the more solvent accessible ones are substituted.

23. Recombinant allergen according to claim 22, wherein one or more amino acid residues of said at least one patch having a solvent accessibility of 20-80 % are substituted.

24. Recombinant allergen according to any one of claims 1-23, wherein 1-5 amino acid residues per 400 Å² in said at least one patch are substituted.

25. Recombinant allergen according to any of claims 1-24, wherein at least one amino acid residue of said B cell epitope is substituted.

26. Recombinant allergen according to any one of claims 1-25, wherein the substitution of one or more amino acid residues in said B cell epitope or said at least one patch is carried out by site -directed mutagenesis.

27. A method of preparing a recombinant allergen according to any one of claims 1-26 comprising the steps of
a) identifying amino acid residues in a naturally occurring allergen which are conserved with more than 70% identity in all known homologous proteins within the taxonomic order from which said naturally occurring allergen originates;
b) defining at least one patch of conserved amino acid residues being coherently connected over at least 400 Å² of the surface of three-dimensional structure of the allergen molecule, said at least one patch comprising at least one B cell epit ope; and
c) substituting at least one surface-exposed amino acid residue, as defined by having a solvent accessibility of at least 20%, in said at least one patch by another amino acid, which does not occur in the same position in the amino acid sequence of any known homologous protein within the taxonomic order from which said naturally occurring allergen originates, while essentially preserving the overall α-carbon backbone tertiary structure of the allergen molecule to obtain a mutated allergen, wherein the specific IgE binding to the mutated allergen is reduced as compared to the binding to said naturally occurring allergen.

28. A method according to claim 27, wherein the amino acid residues of said at least one patch are ranked with respect to solvent accessibility and one or more amino acids among the more solvent accessible ones is/are substituted.

29. A method according to claim 27 or 28, wherein the substitution of one or more amino acid residues in said B cell epitope or said at least one patch is carried out by site-directed mutagenesis.

30. Recombinant allergen according to any of claims 1-26 for use as a pharmaceutical.

31. Pharmaceutical composition comprising a recombinant allergen according to any one of claims 1-26, optionally in combination with a pharmaceutically acceptable carrier and/or excipient, and optionally an adjuvant.

32. A pharmaceutical composition according to claim 31, in the form of a vaccine for treatment or prevention of allergic reactions elicited by a naturally occurring allergen.

33. Process for preparing a pharmaceutical composition according to any one of claims 31-32 comprising mixing at least one recombinant allergen according to any one of claims 1-26 with pharmaceutically acceptable substances and/or excipients.

34. Use of a recombinant allergen as defined in any one of claims 1-26 for the preparation of a medicament for the treatment or prevention of allergic reactions.

## Patentansprüche

1. Nicht-natürlich vorkommendes rekombinantes Mutanten-Allergen, das von einem natürlich vorkommenden Allergen abgeleitet ist, wobei das natürlich vorkommende Allergen wenigstens ein Patch von Aminosäureresten umfasst, die zusammenhängend verbunden sind über mindestens 400 Å² der Oberfläche der dreidimensionalen Struktur des Allergenmoleküls, die eine Lösungsmittelzugänglichkeit von wenigstens 20 % besitzen und die konserviert sind, wie durch die Identität bei mehr als 70 % der Sequenzen von homologen Sequenzen innerhalb derselben taxonomischen Ordnung, zu der das natürlich vorkommende Allergen gehört, definiert, wobei das Patch wenigstens ein B-Zellepitop umfasst, wobei
1) die Mutante wenigstens eine Substitution eines konservierten Aminosäurerests, der in dem wenigstens einen Patch lokalisiert ist, umfasst und wobei der konservierte Aminosäurerest eine Lösungsmittelzugänglichkeit von wenigstens 20 % besitzt, mit einem Aminosäurerest, der nicht in derselben Position in der Aminosäuresequenz irgendeines bekannten homologen Proteins innerhalb der taxonomischen Ordnung, zu der das natürlich vorkommende Allergen gehört, vorkommt,
2) das Mutanten-Allergen im wesentlichen dieselbe Tertiärstruktur des α-Kohlenstoffrückgrats wie das natürlich vorkommende Allergen aufweist, und
3) die spezifische IgE-Bindung an das mutierte Allergen vermindert ist im Vergleich zu der Bindung an das natürlich vorkommende Allergen.

2. Rekombinantes Allergen nach Anspruch 1, wobei das natürlich vorkommende Allergen ein Inhalationsallergen ist.

3. Rekombinantes Allergen nach Anspruch 2, wobei das natürlich vorkommende Allergen ein Pollen-Allergen ist.

4. Rekombinantes Allergen nach Anspruch 3, wobei das natürlich vorkommende Allergen ein Pollen-Allergen ist, das zu der taxonomischen Ordnung der Fagales, Oleales oder Pinales gehört.

5. Rekombinantes Allergen nach Anspruch 4, wobei das natürlich vorkommende Allergen Bet v 1 ist.

6. Rekombinantes Allergen nach Anspruch 5, wobei die Substitution(en) Asp25Gly, (Asn28Thr + Lys32Gln), Glu45Ser, Asn47Ser, Lys55Asn, Thr77Ala, Pro108Gly oder (Asn28Thr, Lys32Gln, Glu45Ser, Pro108Gly) ist/sind.

7. Rekombinantes Allergen nach Anspruch 3, wobei das natürlich vorkommende Allergen ein Pollenallergen ist, das zu der taxonomischen Ordnung der Fagales gehört.

8. Rekombinantes Allergen nach Anspruch 3, wobei das natürlich vorkommende Allergen ein Pollenallergen ist, das zu der taxonomischen Ordnung der Asterales oder Urticales gehört.

9. Rekombinantes Allergen nach Anspruch 1 oder 2, wobei das natürlich vorkommende Allergen ein Hausstaubmilben-Allergen ist.

10. Rekombinantes Allergen nach Anspruch 9, wobei das natürlich vorkommende Allergen ein Milben-Allergen ist, das zu der taxonomischen Gattung der Dermatophagoides gehört.

11. Rekombinantes Allergen nach Anspruch 1 oder 2, wobei das natürlich vorkommende Allergen ein Kakerlaken-Allergen ist.

12. Rekombinantes Allergen nach Anspruch 1 oder 2, wobei das natürlich vorkommende Allergen ein Tier-Allergen ist.

13. Rekombinantes Allergen nach Anspruch 12, wobei das natürlich vorkommende Allergen ein Tier-Allergen ist, das von Katze, Hund oder Pferd stammt.

14. Rekombinantes Allergen nach Anspruch 1, wobei das natürlich vorkommende Allergen ein Tiergift-Allergen ist.

15. Rekombinantes Allergen nach Anspruch 14, wobei das natürlich vorkommende Allergen ein Tiergift-Allergen ist, das zu der taxonomischen Ordnung der Hymenoptera gehört.

16. Rekombinantes Allergen nach Anspruch 15, wobei das natürlich vorkommende Allergen ein Tiergift-Allergen ist, das zu der taxonomischen Ordnung der Vespidae, Apidae und Formicoidae gehört.

17. Rekombinantes Allergen nach Anspruch 16, wobei das natürlich vorkommende Allergen Ves v 5 ist.

18. Rekombinantes Allergen nach Anspruch 17, wobei die Substitution Lys72Ala oder Tyr96Ala ist.

19. Rekombinantes Allergen nach einem der Ansprüche 1-18, wobei die spezifische IgE-Bindung an das mutierte Allergen um wenigstens 5 %, vorzugsweise um wenigstens 10 %, vermindert ist.

20. Rekombinantes Allergen nach einem der Ansprüche 1-19, wobei bei einem Vergleich der Tertiärstrukturen des α-Kohlenstoffrückgrats der mutierten und der natürlich vorkommenden Allergenmoleküle, die mittlere Abweichung des quadratischen Mittelwertes der atomaren Koordinaten unter 2Å liegt.

21. Rekombinantes Allergen nach einem der Ansprüche 1-20, wobei das wenigstens eine Patch Atome von 15-25 Aminosäureresten umfasst.

22. Rekombinantes Allergen nach einem der Ansprüche 1-21, wobei die Aminosäurereste des wenigstens einen Patch bezüglich der Lösungsmittelzugänglichkeit eingeordnet sind und ein oder mehrere Aminosäuren unter den Lösungsmittelzugänglicheren davon substituiert sind.

23. Rekombinantes Allergen nach Anspruch 22, wobei ein oder mehrere Aminosäurereste aus dem wenigstens einen Patch, die eine Lösungsmittelzugänglichkeit von 20-80 % aufweisen, substituiert sind.

24. Rekombinantes Allergen nach einem der Ansprüche 1-23, wobei 1-5 Aminosäurereste pro 400 Å² in dem wenigstens einen Patch substituiert sind.

25. Rekombinantes Allergen nach einem der Ansprüche 1-24, wobei wenigstens ein Aminosäurerest des B-Zellepitops substituiert ist.

26. Rekombinantes Allergen nach einem der Ansprüche 1-25, wobei die Substitution von ein oder mehreren Aminosäureresten in dem B-Zellepitop oder dem wenigstens einen Patch durch ortsgerichtete Mutagenese durchgeführt ist.

27. Methode zum Herstellen eines rekombinierten Allergens nach einem der Ansprüche 1-26, umfassend die folgenden Schritte:
a) Identifizieren von Aminosäureresten in einem natürlich vorkommenden Allergen, die bei mehr als 70 % Identität in allen bekannten homologen Proteinen innerhalb der taxonomischen Ordnung, zu der das natürlich vorkommende Allergen gehört, konserviert sind;
b) Definieren von wenigstens einem Patch von konservierten Aminosäureresten, die zusammenhängend verbunden sind über mindestens 400 Å² der Oberfläche der dreidimensionalen Struktur des Allergenmoleküls, wobei das wenigstens eine Patch mindestens ein B-Zellepitop umfasst; und
c) Substituieren wenigstens eines Oberflächen-exponierten Aminosäurerests, wie durch eine Lösungsmittelzugänglichkeit von wenigstens 20 % definiert, in dem wenigstens einen Patch durch eine andere Aminosäure, die nicht in derselben Position in der Aminosäuresequenz irgendeines bekannten homologen Proteins innerhalb der taxonomischen Ordnung, zu der das natürlich vorkommende Allergen gehört, vorkommt, unter im wesentlichen Erhalten der gesamten Tertiärstruktur des α-Kohlenstoffrückgrats des Allergenmoleküls, um ein mutiertes Allergen zu erhalten, wobei die spezifische IgE-Bindung an das mutierte Allergen vermindert ist im Vergleich zu der Bindung an das natürlich vorkommende Allergen.

28. Methode nach Anspruch 27, wobei die Aminosäurereste des wenigstens einen Patch bezüglich der Lösungsmittelzugänglichkeit eingeordnet sind und eine oder mehrere Aminosäuren unter den Lösungsmittelzugänglicheren davon substituiert ist/sind.

29. Methode nach Anspruch 27 oder 28, wobei die Substitution der ein oder mehreren Aminosäurereste in dem B-Zellepitop oder dem wenigstens einen Patch durch ortsgerichtete Mutagenese durchgeführt wird.

30. Rekombinantes Allergen nach einem der Ansprüche 1-26 zur Verwendung als ein Pharmazeutikum.

31. Pharmazeutische Zusammensetzung, umfassend ein rekombinantes Allergen nach einem der Ansprüche 1-26, wahlweise in Kombination mit einem pharmazeutisch akzeptablen Träger und/oder Exzipienten, und wahlweise einem Adjuvans.

32. Pharmazeutische Zusammensetzung nach Anspruch 31, in der Form eines Impfstoffs zur Behandlung oder Verhütung von allergischen Reaktionen, die durch ein natürlich vorkommendes Allergen hervorgerufen sind.

33. Verfahren zum Herstellen einer pharmazeutischen Zusammensetzung nach einem der Ansprüche 31-32, umfassend das Mischen wenigstens eines rekombinanten Allergens nach einem der Ansprüche 1-26 mit pharmazeutisch akzeptablen Substanzen und/oder Exzipienten.

34. Verwendung eines rekombinanten Allergens, wie in einem der Ansprüche 1-26 definiert, für die Herstellung eines Medikaments zur Behandlung oder Verhütung von allergischen Reaktionen.

## Revendications

1. Allergène recombinant mutant, n'existant pas naturellement, dérivé d'un allergène existant naturellement, l'allergène existant naturellement comprenant au moins une région de résidus aminoacides, qui sont connectés de manière cohérente sur au moins 400 Å² de la surface de la structure en trois dimensions de la molécule d'allergène, qui présentent une accessibilité aux solvants d'au moins 20% et qui sont conservés ainsi que défini comme étant identiques à plus de 70% aux séquences des séquences homologues à l'intérieur du même ordre taxonomique d'où provient l'allergène existant naturellement, cette région comprenant au moins un épitope de lymphocyte B, dans lequel
1) le mutant comprenant au moins une substitution d'un résidu aminoacide conservé situé dans la au moins une région, et le résidu aminoacide conservé ayant une accessibilité aux solvants d'au moins 20%, par un résidu aminoacide qui n'existe pas en la même position dans la séquence aminoacide d'une protéine homologue quelconque connue dans l'ordre taxonomique d'où provient l'allergène existant naturellement,
2) l'allergène mutant ayant essentiellement la même structure tertiaire du squelette de carbones α que les allergènes existant naturellement,
3) la liaison spécifique des IgE à l'allergène muté étant réduite par rapport à la liaison à l'allergène existant naturellement.

2. Allergène recombinant suivant la revendication 1, dans lequel l'allergène existant naturellement est un pneumallergène.

3. Allergène recombinant suivant la revendication 2, dans lequel l'allergène existant naturellement est un allergène de pollen.

4. Allergène recombinant suivant la revendication 3, dans lequel l'allergène existant naturellement est un allergène de pollen provenant de l'ordre taxonomique des Fagales, Oléales ou Pinales.

5. Allergène recombinant suivant la revendication 4, dans lequel l'allergène existant naturellement est Bet v 1.

6. Allergène recombinant suivant la revendication 5, dans lequel la (ou les) substitutions est (ou sont) Asp25Gly, (Asn28Thr + Lys32Gln), Glu45Ser, Asn47Ser, Lys55Asn, Thr77Ala, Pro108Gly ou (Asn28Thr, Lys32Gln, Glu45Ser, Pro108Gly).

7. Allergène recombinant suivant la revendication 3, dans lequel l'allergène existant naturellement est un allergène de pollen provenant de l'ordre taxonomique des Poales.

8. Allergène recombinant suivant la revendication 3, dans lequel l'allergène existant naturellement est un allergène de pollen provenant de l'ordre taxonomique des Astrales ou des Urticales.

9. Allergène recombinant suivant la revendication 1 ou 2, dans lequel l'allergène existant naturellement est un allergène des mites domestiques.

10. Allergène recombinant suivant la revendication 9, dans lequel l'allergène existant naturellement est un allergène de mites provenant du genre taxonomique des Dermatophagoïdes.

11. Allergène recombinant suivant la revendication 1 ou 2, dans lequel l'allergène existant naturellement est un allergène de cafard.

12. Allergène recombinant suivant la revendication 1 ou 2, dans lequel l'allergène existant naturellement est un allergène animal.

13. Allergène recombinant suivant la revendication 12, dans lequel l'allergène existant naturellement est un allergène provenant d'un chat, d'un chien ou d'un cheval.

14. Allergène recombinant suivant la revendication 1, dans lequel l'allergène existant naturellement est un allergène de venin.

15. Allergène recombinant suivant la revendication 14, dans lequel l'allergène existant naturellement est un allergène de venin provenant de l'ordre taxonomique des Hymenoptères.

16. Allergène recombinant suivant la revendication 15, dans lequel l'allergène existant naturellement est un allergène de venin provenant de l'ordre taxonomique des Vespidés, Apidés et Formicoïdés.

17. Allergène recombinant suivant la revendication 16, dans lequel l'allergène existant naturellement est l'allergène Ves v 5.

18. Allergène recombinant suivant la revendication 17, dans lequel la substitution est Lys72Ala ou Tyr96Ala.

19. Allergène recombinant suivant l'une quelconque des revendications 1 à 18, dans lequel la liaison spécifique de IgE à l'allergène muté est réduite d'au moins 5%, de préférence d'au moins 10%.

20. Allergène recombinant suivant l'une quelconque des revendications 1 à 19, dans lequel, lorsque l'on compare les structures tertiaires des squelettes des carbones α du mutant et des molécules d'allergènes existant naturellement, l'écart moyen de la racine carrée des coordonnées atomiques est inférieur à 2 Å.

21. Allergène recombinant suivant l'une quelconque des revendications 1 à 20, dans lequel la au moins une région comprend les atomes de 15 à 25 résidus aminoacides.

22. Allergène recombinant suivant l'une quelconque des revendications 1 à 21, dans lequel les résidus aminoacides de la au moins une région sont classés par ordre d'accessibilité aux solvants, et un ou plusieurs acides aminés parmi ceux qui sont les plus accessibles aux solvants sont substitutés.

23. Allergène recombinant suivant la revendication 22, dans lequel un ou plusieurs résidus aminoacides de la au moins une région ayant une accessibilité aux solvants de 20 à 80% sont substitués.

24. Allergène recombinant suivant l'une quelconque des revendications 1 à 23, dans lequel de 1 à 5 résidus aminoacides par 400 Å₂ dans la au moins une région sont substitués.

25. Allergène recombinant suivant l'une quelconque des revendications 1 à 24, dans lequel un aminoacide au moins de l'épitope de lymphocyte B est substitué.

26. Allergène recombinant suivant l'une quelconque des revendications 1 à 25, dans laquelle la substitution de un ou plusieurs résidus aminoacides dans l'épitope de lymphocyte B ou dans la au moins une région est effectuée par mutagénèse dirigée.

27. Procédé de préparation d'un allergène recombinant suivant l'une quelconque des revendications 1 à 26 comprenant les étapes consistant à :
a) identifier les résidus aminoacides dans un allergène existant naturellement qui sont conservés avec une identité supérieure à 70% dans toutes les protéines homologues connues à l'intérieur de l'ordre taxonomique d'où provient l'allergène existant naturellement ;
b) définir au moins une région de résidus aminoacides conservés connectés de manière cohérente sur au moins 400 Å² de la surface de la structure en trois dimensions de la molécule d'allergène, la au moins une région comprenant au moins un épitope de lymphocyte B ; et
c) substituer un résidu aminoacide au moins qui est exposé à la surface, ainsi que défini comme ayant une accessibilité au solvant d'au moins 20%, dans la au moins une région par un autre acide aminé, qui n'existe pas en cette même position dans la séquence aminoacide d'une protéine homologue quelconque connue dans l'ordre taxonomique d'où provient l'allergène existant naturellement, tout en préservant essentiellement la structure tertiaire globale du squelette de carbones α de la molécule d'allergène afin d'obtenir un allergène muté, dans lequel la liaison spécifique à l'IgE avec l'allergène muté est réduite par rapport à la liaison à l'allergène existant naturellement.

28. Procédé suivant la revendication 27, dans laquelle les résidus aminoacides la au moins une région sont classés par ordre d'accessibilité aux solvants et un ou plusieurs acides aminés parmi ceux qui sont le plus accessibles aux solvants est ou sont substitués.

29. Procédé suivant la revendication 27 ou 28, dans laquelle la substitution du ou des résidus aminoacides à l'intérieur de l'épitope de lymphocyte B ou dans la au moins une région est effectuée par mutagénèse dirigée.

30. Allergène recombinant suivant l'une quelconque des revendications 1 à 26 destiné à une utilisation comme agent pharmaceutique.

31. Composition pharmaceutique comprenant un allergène recombinant suivant l'une quelconque des revendications 1 à 26, facultativement en combinaison avec un véhicule et/ou un excipient acceptables du point de vue pharmaceutique, et facultativement un adjuvant.

32. Composition pharmaceutique suivant la revendication 31, sous la forme d'un vaccin pour le traitement ou la prévention des réactions allergiques induites par un allergène existant naturellement.

33. Procédé de préparation d'une composition pharmaceutique suivant l'une quelconque des revendications 31 à 32, comprenant l'étape consistant à mélanger au moins un allergène recombinant suivant l'une quelconque des revendications 1 à 26 avec des substances et/ou des excipients acceptables du point de vue pharmaceutique.

34. Utilisation d'un allergène recombinant tel que défini dans l'une quelconque des revendications 1 à 26, pour la préparation d'un médicament destiné au traitement ou à la prévention des réactions allergiques.
